(19) Europäisches Patentamt
European Patent Office
Office européen des brevets

(11) EP 0 796 914 B1

(12) **EUROPÄISCHE PATENTSCHRIFT**

(45) Veröffentlichungstag und Bekanntmachung des Hinweises auf die Patenterteilung:
**28.05.2003 Patentblatt 2003/22**

(21) Anmeldenummer: **97104814.5**

(22) Anmeldetag: **20.03.1997**

(51) Int Cl.⁷: **C12N 15/53**, C12N 9/04, C12P 7/22, C12P 7/02, C12N 1/21
// (C12N9/04, C12R1:24), (C12N1/21, C12R1:19)

(54) **Alkohol-Dehydrogenase und deren Verwendung zur enzymatischen Herstellung chiraler Hydroxy-verbindungen**

Alcohol dehydrogenase and its use in the enzymatic production of chiral hydroxy-compounds

Alcool déshydrogénase et son utilisation pour la préparation enzymatique de liaisons hydroxy chirales

(84) Benannte Vertragsstaaten:
**CH DE ES FR GB IT LI**

(30) Priorität: **21.03.1996 DE 19610984**

(43) Veröffentlichungstag der Anmeldung:
**24.09.1997 Patentblatt 1997/39**

(73) Patentinhaber: **Forschungszentrum Jülich GmbH 52425 Jülich (DE)**

(72) Erfinder:
- **Hummel, Werner, Dr. 52455 Titz (DE)**
- **Riebel, Bettina 50968 Köln (DE)**

(56) Entgegenhaltungen:

**EP-A- 0 645 453** **DE-C- 4 014 573**
**FR-A- 2 706 906** **US-A- 5 225 339**

- **W. HUMMEL : "New Alcohol Dehydrogenases for the synthesis of chiral compounds" ADVANCES IN BIOCHEMICAL ENGINEERING/BIOTECHNOLOGY, Bd. 58, 1997, Seiten 145-184, XP000677754**
- **KEINAN E ET AL: "THERMOSTABLE ENZYMES IN ORGANIC SYNTHESIS 7. TOTAL SYNTHESIS OF THE WESTERN CORN ROOTWORM SEX PHEROMONE 8 METHYLDEC-2-YLPROPANOATE USING A TBADH-GENERATED C-2-BIFUNCTIONAL CHIRON." J ORG CHEM 57 (13). 1992. 3631-3636. CODEN: JOCEAH ISSN: 0022-3263, XP000676601**
- **KEINAN E ET AL: "THERMOSTABLE ENZYMES IN ORGANIC SYNTHESIS PART 6. TOTAL SYNTHESIS OF S-LEVO ZEARALENONE USING A TBADH-GENERATED TRIFUNCTIONAL CHIRON." J CHEM SOC PERKIN TRANS I 0 (12). 1991. 3333-3340. CODEN: JCPRB4 ISSN: 0300-922X, XP000676602**
- **HUMMEL W: "REDUCTION OF ACETOPHENONE TO R-DEXTRO PHENYLETHANOL BY A NEW ALCOHOL DEHYDROGENASE FROM LACTOBACILLUS -KEFIR." APPL MICROBIOL BIOTECHNOL 34 (1). 1990. 15-19. CODEN: AMBIDG ISSN: 0175-7598, XP000677515**
- **HUMMEL, WERNER: "Enzyme-catalyzed synthesis of optically pure R(+)-phenylethanol" BIOTECHNOL. LETT. (1990), 12(6), 403-8 CODEN: BILED3;ISSN: 0141-5492, 1990, XP000677511**
- **BRADSHAW C W ET AL: "LACTOBACILLUS -KEFIR ALCOHOL DEHYDROGENASE A USEFUL CATALYST FOR SYNTHESIS." J ORG CHEM 57 (5). 1992. 1532-1536. CODEN: JOCEAH ISSN: 0022-3263, XP000676600**
- **W. HUMMEL ET AL.: "Chiral alcohols by enantioselective enzymatic oxidation." ANNALS OF THE NEW YORK ACADEMY OF SCIENCES. ENZYME ENGINEERING XIII, Bd. 799, 1996, Seiten 713-716, XP000677513**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

SEQUENZPROTOKOLL

(1) ALLGEMEINE ANGABEN:

(i) ANMELDER:

(A) NAME: Boehringer Mannheim GmbH
(B) STRASSE: Sandhoferstr. 116
(C) ORT: Mannheim
(E) LAND: DE
(F) POSTLEITZAHL: 68305
(G) TELEFON: 06217593277
(H) TELEFAX: 06217594457

(ii) BEZEICHNUNG DER ERFINDUNG: Alkohol-Dehydrogenase und deren Verwendung zur enzymatischen Herstellung chiraler Hydroxyverbindungen

(iii) ANZAHL DER SEQUENZEN: 9

(iv) COMPUTER-LESBARE FASSUNG:

(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(2) ANGABEN ZU SEQ ID NO: 1:

(i) SEQUENZKENNZEICHEN:

(A) LÄNGE: 38 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 1:

```
Ser Asn Arg Leu Asp Gly Lys Val Ala Ile Val Thr Gly Gly Thr
 1               5                   10                  15

Leu Gly Ile Gly Leu Ala Ile Ala Thr Lys Phe Val Glu Glu Gly
                20                  25                  30

Ala Lys Val Met Ile Thr Thr Arg
                35
```

(2) ANGABEN ZU SEQ ID NO: 2:

(i) SEQUENZKENNZEICHEN:

(A) LÄNGE: 13 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 2:

```
Val Met Ile Thr Gly Arg His Ser Asp Val Gly Glu Lys
 1               5                   10
```

(2) ANGABEN ZU SEQ ID NO: 3:

(i) SEQUENZKENNZEICHEN:

(A) LÄNGE: 15 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 3:

```
Asp Tyr Asp Val Arg Val Asn Thr Val His Pro Gly Tyr Ile Lys
 1               5                   10                  15
```

(2) ANGABEN ZU SEQ ID NO: 4:

(i) SEQUENZKENNZEICHEN:

(A) LÄNGE: 16 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 4:

```
Phe Ala Thr Gly Ser Glu Phe Val Val Asp Gly Gly Tyr Thr Ala Gln
 1               5                  10                  15
```

(2) ANGABEN ZU SEQ ID NO: 5:

(i) SEQUENZKENNZEICHEN:

(A) LÄNGE: 33 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 5:

```
ATGTCAAACC GTTTRGATGG YAAGGTRGCR ATY
33
```

(2) ANGABEN ZU SEQ ID NO: 6:

(i) SEQUENZKENNZEICHEN:

(A) LÄNGE: 36 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 6:

```
CTACTATTGY GCAGTGTARC CRCCATCYAC YACGAA
36
```

(2) ANGABEN ZU SEQ ID NO: 7:

(i) SEQUENZKENNZEICHEN:

(A) LÄNGE: 756 Basenpaare
(B) ART: Nucleotid
(C) STRANGFORM: Doppelstrang
(D) TOPOLOGIE: linear

(ii) ART DES MOLEKÜLS: Genom-DNA

(ix) MERKMAL:

(A) NAME/SCHLÜSSEL: CDS
(B) LAGE:1..756

(xi) SEQUENZBESCHREIBUNG: SEQ ID NO: 7:

```
ATG TCT AAC CGT TTG GAT GGT AAG GTA GCA ATC ATT ACA GGT GGT
45
Met Ser Asn Arg Leu Asp Gly Lys Val Ala Ile Ile Thr Gly Gly
 1               5                  10                  15

ACG TTG GGT ATC GGT TTA GCT ATC GCC ACG AAG TTC GTT GAA GAA
90
Thr Leu Gly Ile Gly Leu Ala Ile Ala Thr Lys Phe Val Glu Glu
                20                  25                  30

GGG GCT AAG GTC ATG ATT ACC GGC CGG CAC AGC GAT GTT GGT GAA
135
Gly Ala Lys Val Met Ile Thr Gly Arg His Ser Asp Val Gly Glu
                35                  40                  45

AAA GCA GCT AAG AGT GTC GGC ACT CCT GAT CAG ATT CAA TTT TTC
180
Lys Ala Ala Lys Ser Val Gly Thr Pro Asp Gln Ile Gln Phe Phe
                50                  55                  60

CAA CAT GAT TCT TCC GAT GAA GAC GGC TGG ACG AAA TTA TTC GAT
225
Gln His Asp Ser Ser Asp Glu Asp Gly Trp Thr Lys Leu Phe Asp
                65                  70                  75

GCA ACG GAA AAA GCC TTT GGC CCA GTT TCT ACA TTA GTT AAT AAC
270
Ala Thr Glu Lys Ala Phe Gly Pro Val Ser Thr Leu Val Asn Asn
                80                  85                  90

GCT GGG ATC GCG GTT AAC AAG AGT GTC GAA GAA ACC ACG ACT GCT
315
Ala Gly Ile Ala Val Asn Lys Ser Val Glu Glu Thr Thr Thr Ala
                95                 100                 105
```

GAA TGG CGT AAA TTA TTA GCC GTC AAC CTT GAT GGT GTC TTC TTC
360
Glu Trp Arg Lys Leu Leu Ala Val Asn Leu Asp Gly Val Phe Phe
110 115 120

GGT ACC CGA TTA GGG ATT CAA CGG ATG AAG AAC AAA GGC TTA GGG
405
Gly Thr Arg Leu Gly Ile Gln Arg Met Lys Asn Lys Gly Leu Gly
125 130 135

GCT TCC ATC ATC AAC ATG TCT TCG ATC GAA GGC TTT GTG GGT GAT
450
Ala Ser Ile Ile Asn Met Ser Ser Ile Glu Gly Phe Val Gly Asp
140 145 150

CCT AGC TTA GGG GCT TAC AAC GCA TCT AAA GGG GCC GTA CGG ATT
495
Pro Ser Leu Gly Ala Tyr Asn Ala Ser Lys Gly Ala Val Arg Ile
155 160 165

ATG TCC AAG TCA GCT GCC TTA GAT TGT GCC CTA AAG GAC TAC GAT
540
Met Ser Lys Ser Ala Ala Leu Asp Cys Ala Leu Lys Asp Tyr Asp
170 175 180

GTT CGG GTA AAC ACT GTT CAC CCT GGC TAC ATC AAG ACA CCA TTG
585
Val Arg Val Asn Thr Val His Pro Gly Tyr Ile Lys Thr Pro Leu
185 190 195

GTT GAT GAC CTA CCA GGG GCC GAA GAA GCG ATG TCA CAA CGG ACC
630
Val Asp Asp Leu Pro Gly Ala Glu Glu Ala Met Ser Gln Arg Thr
200 205 210

```
AAG ACG CCA ATG GGC CAT ATC GCT GAA CCT AAC GAT ATT GCC TAC
675
Lys Thr Pro Met Gly His Ile Ala Glu Pro Asn Asp Ile Ala Tyr
                215                 220                 225

ATC TGT GTT TAC TTG GCT TCT AAC GAA TCT AAA TTT GCA ACG GGT
720
Ile Cys Val Tyr Leu Ala Ser Asn Glu Ser Lys Phe Ala Thr Gly
                230                 235                 240

TCT GAA TTC GTA GTT GAC GGT GGC TAC ACT GCT CAA
756
Ser Glu Phe Val Val Asp Gly Gly Tyr Thr Ala Gln
                245                 250
```

(2) ANGABEN ZU SEQ ID NO: 8:

(i) SEQUENZKENNZEICHEN:

(A) LÄNGE: 252 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 8:

Met Ser Asn Arg Leu Asp Gly Lys Val Ala Ile Ile Thr Gly Gly
1 5 10 15

Thr Leu Gly Ile Gly Leu Ala Ile Ala Thr Lys Phe Val Glu Glu
20 25 30

Gly Ala Lys Val Met Ile Thr Gly Arg His Ser Asp Val Gly Glu
35 40 45

Lys Ala Ala Lys Ser Val Gly Thr Pro Asp Gln Ile Gln Phe Phe
50 55 60

Gln His Asp Ser Ser Asp Glu Asp Gly Trp Thr Lys Leu Phe Asp
65 70 75

Ala Thr Glu Lys Ala Phe Gly Pro Val Ser Thr Leu Val Asn Asn
80 85 90

Ala Gly Ile Ala Val Asn Lys Ser Val Glu Glu Thr Thr Thr Ala
95 100 105

Glu Trp Arg Lys Leu Leu Ala Val Asn Leu Asp Gly Val Phe Phe
110 115 120

Gly Thr Arg Leu Gly Ile Gln Arg Met Lys Asn Lys Gly Leu Gly
125 130 135

Ala Ser Ile Ile Asn Met Ser Ser Ile Glu Gly Phe Val Gly Asp
140 145 150

Pro Ser Leu Gly Ala Tyr Asn Ala Ser Lys Gly Ala Val Arg Ile
155 160 165

```
Met Ser Lys Ser Ala Ala Leu Asp Cys Ala Leu Lys Asp Tyr Asp
                170                 175                 180

Val Arg Val Asn Thr Val His Pro Gly Tyr Ile Lys Thr Pro Leu
                185                 190                 195

Val Asp Asp Leu Pro Gly Ala Glu Glu Ala Met Ser Gln Arg Thr
                200                 205                 210

Lys Thr Pro Met Gly His Ile Ala Glu Pro Asn Asp Ile Ala Tyr
                215                 220                 225

Ile Cys Val Tyr Leu Ala Ser Asn Glu Ser Lys Phe Ala Thr Gly
                230                 235                 240
Ser Glu Phe Val Val Asp Gly Gly Tyr Thr Ala Gln
                245                 250
```

(2) ANGABEN ZU SEQ ID NO: 9:

(i) SEQUENZKENNZEICHEN:

(A) LÄNGE: 251 Aminosäuren
(B) ART: Aminosaure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 9:

```
Thr Asp Arg Leu Lys Gly Lys Val Ala Ile Val Thr Gly Gly Thr
 1               5                  10                  15

Leu Gly Ile Gly Leu Ala Ile Ala Asp Lys Phe Val Glu Glu Gly
                20                  25                  30
```

Ala Lys Val Val Ile Thr Gly Arg His Ala Asp Val Gly Glu Lys
35 40 45

Xaa Xaa Xaa Xaa Xaa Xaa Xaa Xaa Xaa Xaa Xaa Xaa Xaa Xaa Xaa
50 55 60

Xaa Xaa Xaa Xaa Xaa Xaa Xaa Xaa Xaa Xaa Xaa Leu Phe Asp Ala
65 70 75

Thr Glu Glu Ala Phe Gly Pro Val Thr Thr Val Val Asn Asn Ala
80 85 90

Gly Ile Ala Val Asn Lys Ser Val Glu Asp Thr Thr Thr Glu Glu
95 100 105

Trp Arg Lys Leu Leu Ser Val Asn Leu Asp Gly Val Phe Phe Gly
110 115 120

Thr Arg Leu Gly Ile Gln Ala Met Lys Xaa Xaa Gly Leu Gly Ala
125 130 135

Ser Ile Ile Asn Met Ser Ser Ile Glu Gly Phe Val Gly Asp Pro
140 145 150

Thr Leu Gly Ala Tyr Asn Ala Ser Lys Gly Ala Val Arg Ile Met
155 160 165

Ser Lys Ser Ala Ala Leu Asp Cys Ala Leu Lys Asp Tyr Asp Val
170 175 180

Arg Val Asn Thr Val His Pro Gly Tyr Ile Lys Thr Pro Leu Val
185 190 195

Asp Asp Leu Glu Gly Ala Glu Glu Met Met Ser Gln Arg Thr Lys
200 205 210

```
Thr Pro Met Gly His Ile Gly Glu Pro Asn Asp Ile Ala Tyr Ile
                215                 220                 225

Cys Val Tyr Leu Ala Ser Asp Glu Thr Lys Phe Ala Thr Gly Ala
                230                 235                 240
Glu Phe Val Val Asp Gly Gly Tyr Thr Ala Gln
                245                 250
```

## Beschreibung

Die vorliegende Erfindung betrifft neue enantioselektive Alkohol-Dehydrogenasen (ADH) aus Mikroorganismen, wie beispielsweise der Lactobacillus-Art, insbesondere aus Lactobacillus brevis. Die neuen Enzyme sind besonders vorteilhaft zur Reduktion von organischen Ketoverbindungen zu den entsprechenden Hydroxyverbindungen geeignet, wobei diese Reduktionen enantioselektiv zu den entsprechenden R-Verbindungen führen. Der Enantiomerenüberschuß, der sich berechnet nach

$$ee\ (\%) = (R)\text{-Produkt} - (S)\text{-Produkt} / (R)\text{-Produkt} + (S)\text{-Produkt} \times 100,$$

beträgt dabei in aller Regel mehr als 95%. S-Hydroxy-Verbindungen waren bei Verwendung der erfindungsgemäßen ADH nicht nachweisbar. Aufgrund des breiten Substratspektrums können mit dem erfindungsgemäßen Enzym beispielsweise chirale Alkohole, chirale Hydroxyester (beispielsweise $\alpha$- und $\beta$-Hydroxyester) oder auch Hydroxysäuren hergestellt werden.

Optisch aktive Hydroxyverbindungen sind wertvolle chirale Bausteine, die mit klassischen chemischen Verfahren schwer zugänglich sind. Zur Herstellung chiraler Verbindungen werden daher in der Regel biotechnologische Verfahren in Betracht gezogen, entweder unter Verwendung ganzer Mikroorganismen-Zellen oder mittels isolierter Enzyme. Wie beispielsweise die Veröffentlichung von F. Aragozzini et al. (Appl. Microbiol. Biotechnol. (1986) 24, 175-177 zeigt, ergeben Verfahren mit ganzen Zellen häufig niedrige Ausbeuten, einen nur geringen Enantiomerenüberschuß (d.h. niedrige ee-Werte) und lange Umsetzungszeiten, so daß Enzyme, die in gereinigter und konzentrierter Form eingesetzt werden können, vorteilhafter sind. Für chirale Hydroxyverbindungen, wie beispielsweise Alkohole, bieten sich Alkohol-Dehydrogenasen an, die die prochirale Verbindung mit Hilfe eines Coenzyms (häufig NADH oder NADPH) reduzieren. Diese Reaktionen sind in der Regel hoch enantioselektiv. Die bisher verfügbaren Alkohol-Dehydrogenasen (ADH) führen alle zu S-Alkoholen, teilweise ist bei diesen Enzymen das Substratspektrum relativ schmal (Hefe-ADH, Pferdeleber-ADH). Eine NADP-abhängige Alkohol-Dehydrogenase aus Lactobacillus kefir ist in DE 40 14 573 C1 beschrieben. Diese führt zu R-Alkoholen. Es hat sich allerdings gezeigt, daß dieses Enzym relativ instabil ist, eine Reinigung bis zum homogenen Enzym war somit nur unter großen Verlusten (>98%) möglich.

Durch intensives Screening konnte nun überraschenderweise eine R-spezifische Alkohol-Dehydrogenase mit deutlich höherer Stabilität gefunden werden. Bestimmt man die thermische Desaktivierung dieses Enzyms im Vergleich zu dem ähnlichsten vorbekannten ADH-Enzym, zeigt sich, daß das vorbekannte Enzym bei bereits 45°C zu 50% inaktiviert wird, während das erfindungsgemäße Enzym erst bei Temperaturen von ca. 65°C zu 50% inaktiviert ist. Die höhere Thermostabilität bedeutet zudem eine höhere Lagerstabilität bzw. Stabilität unter bestimmten Reaktionsbedingungen.

Ein entsprechend stabiles R-ADH-Enzym konnte insbesondere in Mikroorganismen der Gattung Lactobacillus, wie L. brevis und der Untergruppe Betabacterium (Gruppe A) gefunden werden. Bisher war von keinem Mikroorganismus bzw. Organismus bekannt, daß er eine stabile R-spezifische Alkohol-Dehydrogenase aufweist. Mit Hilfe eines Antikörpers gegen das L. kefir ADH-Protein konnte nun gezeigt werden, daß alle Lactobacillen der Untergruppe Betabacterium (Gruppe A) ein entsprechendes mit dem Antikörper reagierendes Protein besitzen. Eine besonders gute enzymatische Aktivität ist dagegen insbesondere bei Lactobacillus brevis nachweisbar. Die anderen Stämme dieser Gruppe besitzen jedoch ebenfalls ein für solche Umsetzungen prinzipiell geeignetes Enzym, auch wenn sie unter den gegebenen Anzucht- und Testbedingungen etwas geringere Aktivität zeigen. Lactobacillen anderer Untergruppen (Thermobacterium, IA, Streptobacterium IB, Betabacterium Gruppe B) zeigen dagegen weder eine Reaktion im Antikörper-Test, noch verfügen sie über entsprechende enzymatische Aktivität.

Das stabile, aus L. brevis erhältliche Enzym konnte bis zur Homogenität gereinigt werden. Entsprechende Daten zur Proteinsequenz wurden mit dem gereinigten Enzym ermittelt. Ein Sequenz-Vergleich der N-terminalen Aminosäuren zeigt, daß zwar größere Übereinstimmungen zwischen dem erfindungsgemäßen stabilen Enzym und ADHs anderen mikrobiellen Ursprungs vorliegen, einige Aminosäuren jedoch ausgetauscht sind.

Die nachfolgend aufgeführte biochemische Charakterisierung zeigt weitere Unterschiede des erfindungsgemäßen Enzyms gegenüber entsprechend vorbekannten Enzymen, z.B. bezüglich der relativen Aktivitäten gegenüber Ketonen. Die maximale Stabilität der erfindungsgemäßen ADH liegt beispielsweise bei einem pH-Wert von ca. 9,0. Eine gute Stabilität weist das Enzym darüber hinaus bei ca. pH 5,5 auf. Dies gilt beispielsweise in einem 50 mM MES-Puffer (MES = 2-Morpholinoethansulfonsäure). Im selben Puffersystem zeigt das erfindungsgemäße Enzym nach ca. 30 Minuten bei einer Temperatur zwischen 25°C bis 60°C noch eine Restaktivität von über 95%. Ein Stabilitätsmaximum weist das Enzym bei ca. 40°C auf. Ferner besitzt das Enzym ein Aktivitätsmaximum bei ca. 50°C.

Das erfindungsgemäße Enzym unterscheidet sich zudem auf Aminosäuresequenzebene von vorbekannten ADH-Enzymen. Beispielsweise weist das N-terminale Ende fünf Aminosäureaustausche auf einer Gesamtlänge von 38 Aminosäuren (AS) auf, was einen AS-Unterschied von über 12% bedeutet. Sämtliche Vergleiche zeigen somit, daß

es vorteilhaft ist, für entsprechende Anwendungen die erfindungsgemäße Alkohol-Dehydrogenase, insbesondere die aus Lactobacillus brevis zu verwenden.

Ein weiterer Gegenstand der Erfindung betrifft die Gewinnung des erfindungsgemäßen Enzyms aus geeigneten Mikroorganismen. Gute Enzymausbeuten wurden mit einem Stamm von Lactobacillus brevis erzielt, der unter der Nummer DSM 20054 am 06.06.1972 bei der Deutschen Sammlung von Mikroorganismen und Zellkulturen, Braunschweig, hinterlegt wurde und frei zugänglich ist.

Das Verfahren zur Gewinnung bzw. Reinigung des ADH-Enzyms läuft im wesentlichen über folgende Verfahrensschritte: Aufschluß der kultivierten Zellen, beispielsweise mechanisch durch Glasperlen, Durchführung einer hydrophoben Chromatographie bzw. Interaktionschromatographie und anschließender Anionenaustausch- sowie Affinitätschromatographie. Es hat sich insbesondere als vorteilhaft erwiesen, wenn sämtliche Homogenisation- bzw. Elutionspuffer ca. 0,5 bis 5 mM Magnesium enthalten. Als besonders vorteilhaft hat sich hierbei eine $Mg^{2+}$-Konzentration von ca. 1 mM erwiesen. Auf diese Weise wird das Enzym mit einer spezifischen Aktivität von mindestens 400 U/mg, in vielen Fällen bis zu 500 U/mg gewonnen.

Das erfindungsgemäße Enzym kann außerdem durch rekombinante Verfahren, d.h. durch Expression entsprechender für das Enzym codierender DNA in einem geeigneten prokaryotischen oder eukaryotischen Stamm hergestellt werden. Insbesondere ist ein System, welches auf einem ADH-Strukturgen aus Lactobacillus brevis in Escherichia coli basiert, geeignet. Dabei hat sich überraschenderweise als besonders vorteilhaft erwiesen, daß die Alkoholdehydrogenase ausschließlich als lösliches und aktives Protein exprimiert wird.

Ein weiterer Gegenstand der Erfindung ist ein Verfahren zur enantioselektiven Reduktion von organischen Ketoverbindungen gemäß der allgemeinen Formel (I)

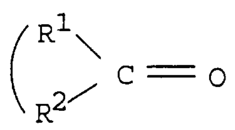

(I)

wobei R' und $R^2$, verschieden oder identisch, Wasserstoff, zusammen oder jeweils, ein geradkettiger oder verzweigter Alkyl- oder Alkenylrest, ein Aryl- oder Arylenylrest, jeweils aus 1 bis 20 C-Atomen bestehend, die durch ein oder mehrere Halogenatome, Nitro-, Hydroxyl- oder Alkoxyreste, wobei die Alkoxyreste 1 bis 20 C-Atome aufweisen, eine gegebenenfalls substituierte Stickstoff-, Sauerstoff- oder Schwefelheterocyclen substituiert sind, oder ein gegebenenfalls substituierter polykondensierter gesättigter und/oder ungesättigter aromatischer Rest sein können, welches dadurch gekennzeichnet ist, daß man eine Ketoverbindung bzw. ein entsprechendes Gemisch mit einer erfindungsgemäßen mikrobiellen Alkohol-Dehydrogenase behandelt. Hierbei kann sowohl das erfindungsgemäße Enzym als solches, als auch eine Kultur von Mikroorganismen, welche dieses Enzym produzieren, bzw. das Enzym enthaltene Zellen verwendet werden. Die Reaktion erfolgt vorzugsweise in einem wäßrigen Puffer, z.B. Kaliumphosphat-, Tris/HCl- oder Triethanolamin (TEA)-Puffer in Gegenwart von Magnesiumionen, bei einem pH-Wert von 5 bis 10, vorzugsweise pH 6 bis 9, und einer Temperatur von 10° bis 70°C, vorzugsweise von 30° bis 60°C. Ferner sind in dem Reaktionsansatz ein entsprechendes Coenzym, wie z.B. NAD(P)H sowie ein zur Regenerierung von oxidiertem Coenzym geeignetes Mittel, wie beispielsweise Isopropanol vorhanden. Wenn ein ausreichender Umsatz, wie vorzugsweise ein 50%-iger Umsatz erreicht ist, wird die Reaktion abgebrochen, vorzugsweise durch direkt anschließende Extraktion, wie beispielsweise in Chloroform. Die Reaktion kann jedoch auch durch Erniedrigung des pH-Wertes, durch Erhitzen oder Zugabe eines geeigneten Enzyminhibitors abgebrochen werden. Anschließend daran wird die erhaltene enantiomerenreine R-Hydroxyverbindung mit einem mit Wasser nicht mischbaren organischen Lösungsmittel extrahiert und gemäß bekannten Verfahren durch Chromatographie oder Destillation von nicht umgesetzter Ketoverbindung bzw. anderen Ausgangsverbindungen bzw. Komponenten abgetrennt. Die Enantiomerenreinheit wird zweckmäßigerweise über GC und/oder HPLC an einem chiralen Säulenmaterial oder über ein Polarimeter bestimmt. Insbesondere hat sich das erfindungsgemäße Verfahren zur Reduktion von Ketonen, Ketoestern wie α-, β- oder γ-Ketoestern, bzw. cyclischen Aryl- und Alkylestern, bevorzugt von solchen mit durch beispielsweise Halogen- bzw. Alkyl- substituierten Resten, als geeignet erwiesen. Besonders gute Ergebnisse konnten bei Acetophenonderivaten, Methylcyclohexanonen, bestimmten Diketonen, wie 2,4-Pentandion, verschiedenen Acetessigsäureestern sowie Alkylestern, wie beispielsweise Ethylpyruvat erzielt werden.

Ein weiterer Gegenstand der Erfindung ist ein Verfahren zur Herstellung einer enantioselektiven Hydroxyverbindung gemäß der allgemeinen Formel (II)

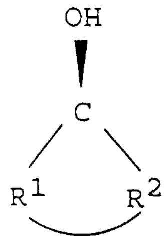

(II)

wobei $R^1$ und $R^2$ jeweils die oben angegebenen Bedeutungen haben, welches dadurch gekennzeichnet ist, daß man ein racemisches Gemisch einer organischen Hydroxyverbindung mit einer erfindungsgemäßen mikrobiellen Alkohol-Dehydrogenase bzw. die ADH enthaltene Zellen in einem wäßrigen Puffer bei einem pH-Wert von 5 bis 10, vorzugsweise pH 6 bis 9 und einer Temperatur von 10° bis 70°C, vorzugsweise von 30° bis 60°C, behandelt und anschließend die erhaltene enantiomerenreine S-Hydroxyverbindung isoliert. Ansonsten gelten die oben genannten bzw. dem Fachmann für entsprechende Reaktionen bekannten Bedingungen.

Die erfindungsgemäße ADH kann für die beschriebenen Reaktionen entweder vollständig oder teilweise gereinigt bzw. in Zellen enthaltend eingesetzt werden. Die Zellen können dabei nativ, permeabilisiert oder lysiert vorliegen.

Die Erfindung wird in nachfolgenden Beispielen näher erläutert:

Beispiel 1:

Screening nach R -Alkohol-Dehydrogenasen unter Stämmen der Gattung Lactobacillus

Die Gattung Lactobacillus wird aufgrund von physiologischen Merkmalen taxonomisch in drei Untergruppen aufgeteilt: Thermobacterium, Streptobacterium und Betabacterium, letztere noch einmal in die beiden Untergruppen A und B. Mit einem Antikörper gegen die Alkohol-Dehydrogenase aus Lactobacillus kefir wurden Typstämme aller Untergruppen getestet, ob sie eine Reaktion gegen den Antikörper zeigen. Überraschenderweise zeigten alle getesteten Stämme der Untergruppe A von Betabacterium Reaktivität, alle anderen Stämme waren dagegen inaktiv. Rohextrakte der Stämme der Untergruppe A von Betabacterium wurden daraufhin im Enzymtest getestet, inwieweit auch enzymatische Aktivität vorliegt. Die Ergebnisse sind in Tabelle 1 zusammengefaßt. Diese zeigt, daß unter den gegebenen Anzucht- und Testbedingungen mehrere Stämme dieser Untergruppe keine oder nur schwache Enzymaktivität zeigen, obwohl der Antikörpertest positiv ausfiel. Auffallend war allerdings eine besonders hohe Enzymaktivität an ADH in Proben von Lactobacillus brevis.

Tabelle 1

| Vorkommen von R-Alkohol-Dehydrogenase in Stämmen der Gattung Lactobacillus | | |
|---|---|---|
| Stamm | Reaktivität im Antikörper-Test | Enzymaktivität (Acetophenon-Reduktion) [U/ml] |
| Thermobacterium | | |
| L. acidophilus | 0 | 0 |
| L. helveticus | 0 | 0 |
| L. bulgaricus | 0 | 0 |
| L. delbrueckii | 0 | 0 |
| L. salivarius | 0 | 0 |
| Streptobacterium: | | |
| L. casei | 0 | 0 |
| L. plantarum | 0 | 0 |
| L. alimentarius | 0 | 0 |
| L. curvatus | 0 | 0 |
| L. coryneformis | 0 | 0 |
| L. farciminis | 0 | 0 |

Tabelle 1 (fortgesetzt)

| Vorkommen von R-Alkohol-Dehydrogenase in Stämmen der Gattung Lactobacillus | | |
|---|---|---|
| Stamm | Reaktivität im Antikörper-Test | Enzymaktivität (Acetophenon-Reduktion) [U/ml] |
| Betabacterium Gruppe A: | | |
| L. kefir | + | 87,0 |
| L. brevis | + | 93,0 |
| L. cellobiosus | + | 0,9 |
| L. fermentum | + | 0,2 |
| L. viridescens | + | 0,2 |
| L. confusus | + | 0,3 |
| L. buchneri | 0 | 0,8 |
| Betabacterium Gruppe B: | | |
| L. hilgardii | 0 | 0 |
| L. fructivorans | 0 | 0 |

Beispiel 2:

Gewinnung und Reinigung von Alkohol-Dehydrogenase aus Lactobacillus brevis

A. Kultivierung von Lactobacillus brevis

Zur Enzymgewinnung wurde Lactobacillus brevis in folgendem Medium angezogen (Angaben g/L):

Glucose (20), Hefeextrakt (5), Trypton (10), Fleischextrakt (5), Di-Ammoniumhydrogencitrat (2), Natriumacetat (5), Magnesiumsulfat (0.1), Mangansulfat (0.05), Di-Kaliumhydrogenphosphat (2).

Die Lösung wurde auf 1L aufgefüllt und der pH-Wert auf 6.5 eingestellt, anschließend wurde das Medium bei 121°C (2 bar) für 10 min sterilisiert. Der Stamm wird ohne weitere Sauerstoffzufuhr oder pH-Regulierung bei 30°C kultiviert.

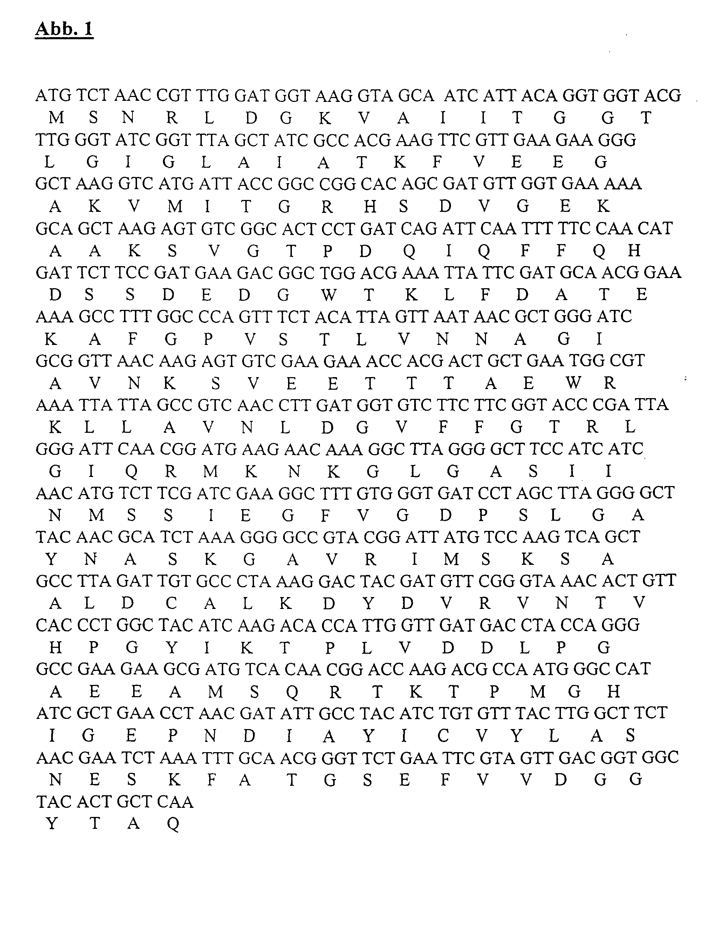

Im 10L Maßstab wurde das Medium im Fermenter nach Sterilisation und Temperierung auf 30°C mit einer 4%igen Vorkultur ($OD_{660}$ 0.35) beimpft. Für diesen Ansatz wurde beispielhaft der Verlauf des Zellwachstums und der Enzymaktivität über die Zeit bestimmt, indem zu bestimmten Zeitpunkten Proben entnommen wurden, die dann auf $OD_{660}$, Frischgewicht und Enzymaktivität nach Aufschluß der Zellen untersucht wurden. In Abb. 1 ist ein solcher Verlauf dargestellt, die Aktivität der Alkohol-Dehydrogenase aus L. brevis zeigt nach Erreichen der stationären Phase des Wachstums den maximalen Aktivitätswert, der nur für 1.5 h gehalten wird.

Im 220L Maßstab wurde der Organismus unter den gleichen Bedingungen kultiviert, nach 13 h bei einem pH-Wert von 5.3 und einer $OD_{660}$ von 2,2 wurden nach Zentrifugation 700 g Zellmasse gewonnen. Die Zellmasse kann bei -20°C gelagert werden, ohne daß über mehrere Monate ein Aktivitätsverlust meßbar ist. Figur 1 zeigt das Wachstum (dargestellt als optische Dichte) und die Enzymbildung (U/G Zellen und U/mg Protein) in Abhängigkeit von der Fermentationszeit.

B. Enzymisolierung (Rohextraktherstellung)

Die Enzymfreisetzung aus den Zellen wurde durch Naßvermahlung mit Hilfe von Glasperlen (0.3 mm) erreicht, kann aber auch durch jede andere Methode zum Aufschluß von Bakterienzellen erzielt werden. Die Bakterienmasse wird mit 0.1 M Acetatpuffer pH 4.0 + 1 mM $MgCl_2$ unter Zusatz von Antischaummittel (Polypropylenglycol) zu einer 40%igen Suspension verdünnt. Diese wurde unter Zugabe von Glasperlen im Verhältnis von 1:2 im Disintegrator (Fa. IMA) bei 4000 rpm einem 20minütigen Aufschluß bei ständiger Kühlung unterzogen. 8 g Bakterien ergaben 10 ml Rohextrakt mit einer Volumenaktivität von 40 U/ml und einem Proteingehalt von ca. 3 mg/ml. Der Enzymtest enthält 970 µl Triethanolaminpuffer (100 mM; pH 7.0; mit 11 mM Acetophenon), 20 µl NADPH (Endkonzentration 0.19 mM) und Enzymlösung.

Definition der Enzymeinheiten: 1 U entspricht der Enzymmenge, die benötigt wird, um 1 µmol Substrat (Acetophenon) pro 1 min umzusetzen.

C. Reinigung der Alkohol-Dehydrogenase aus Lactobacillus brevis

Das Enzym kann über hydrophobe Interaktions-Chromatographie mit nachfolgenden Anionenaustauschchromatographie und Affinitätschromatographie bis zur Homogenität gereinigt werden. Dabei empfiehlt sich ein Zusatz von $Mg^{2+}$-Ionen von ca. 1 mM zu allen Puffern. Bei Entzug dieser Ionen kann das Enzym irreversibel geschädigt werden.

1. Hydrophobe Interaktionschromatographie

5 ml eines Rohextrakts (entsprechend Beispiel 2 B) werden über kleine Gelfiltrationssäulen (PD10, Pharmacia) in 50 mM Triethanolaminpuffer pH 7.0 mit 1 mM $MgCl_2$ und 0.6 M $(NH_4)_2SO_4$ umgepuffert und auf eine Phenyl-Sepharose CL-6B (Fa. Pharmacia, Freiburg, Deutschland) aufgetragen. Die Säule ist mit 50 mM Triethanolaminpuffer pH 7.0, 1 mM $MgCl_2$ und 0.6 M $(NH_4)_2SO_4$ äquilibriert worden. Nach Auftrag und Spülen der Säule mit dem Äquilibrierungspuffer wird das Enzym mit einem fallenden linearen Salzgradienten (6 bis 0 M $(NH_4)_2SO_4$, Fluß 1 ml/min) bei 0.36 M $(NH_4)_2SO_4$ eluiert. Die aktiven Fraktionen werden zusammengefaßt und auf eine Konzentration von 1.2 M $(NH_4)_2SO_4$ gebracht. Diese Proteinlösung wird auf eine Octyl-Sepharosesäule aufgetragen, die mit 50 mM Triethanolaminpuffer pH 7.0, 1 mM $MgCl_2$ und 1.2 M $(NH_4)_2SO_4$ äquilibriert wurde. Nach Auftrag der Proteinlösung und anschließendem Spülen der Säule wird das Enzym mit einem fallenden linearen Salzgradienten (1.2 bis 0 M $(NH_4)_2SO_4$, Fluß 1 ml/min) bei 1.0 M $(NH_4)_2SO_4$ eluiert. Die erzielte Anreicherung ist in Tabelle 2 zusammengefaßt.

2. Anionenaustauschchromatographie

Die Fraktionen mit der höchsten Aktivität der letzten oben verwendeten Säule werden über Gelfiltrationssäulen (PD10, Pharmacia) in 50 mM Tris/HCl-Puffer pH 9.0 mit 1 mM $MgCl_2$ umgepuffert und auf eine ebenso äquilibrierte Mono Q-Säule (Fluß 1 ml/min; Druck 1.5 MPA; FPLC-Chromatographiesystem; Fa. Pharmacia, Freiburg, Deutschland) aufgetragen. Nach erfolgtem Spülen der Säule wird die Alkohol-Dehydrogenase mit einem linearen Salzgradienten von 0 bis 0.6 M NaCl eluiert, wobei das Enzym bei 0.36 M NaCl erscheint.

3. Affinitätschromatographie

Die aktiven Fraktionen werden über Gelfiltrationssäulen (PD10, Fa. Pharmacia), die mit 50 mM Morpholinethansulfonsäurepuffer pH 5.5 und 1 mM $MgCl_2$ äquilibriert wurden, umgepuffert und auf eine ebenso äquilibrierte 2',5'-AMP-Sepharosesäule (Fa. Pharmacia) aufgetragen. Anschließend wird mit 100 mM NaCl, gelöst im gleichen Puffer, gespült. Anschließend erfolgt die Elution mit einem linearen NADP-Gradienten von 0 bis 10 mM NADP. Die Dehydrogenase eluiert bei 3.33 mM NADP. Die Chromatographie wurde mit einer Flußrate von 0.25 ml/min durchgeführt. Die komplette Reinigung des Enzyms ist in Tabelle 2 zusammengefaßt.

Tabelle 2

| Reinigung der Alkohol-Dehydrogenase aus Lactobacillus brevis | | | |
|---|---|---|---|
| Reinigungsschritt | Aktivität [U/ml] | spez. Akt. [U/mg] | Ausbeute [%] |
| Rohextrakt | 40.8 | 14.78 | 100 |
| Phenylsepharose | 17.21 | 45.23 | 42 |
| Octylsepharose | 4.6 | 92 | 8 |
| Mono Q | 2.47 | 183 | 4 |
| 2',5'-AMP-Seph. | 11.74 | 489 | 3 |

D. Reinigung der Alkohol-Dehydrogenase aus Lactobacillus kefir

Lactobacillus kefir besitzt eine intrazelluläre NADP-abhängige Alkohol-Dehydrogenase (DE 40 14 573 C1), die in einigen biochemischen Eigenschaften (Produktion von R-Alkoholen, Coenzym-Spezifität) der Alkohol-Dehydrogenase aus Lactobacillus brevis ähnelt. Um die Unterschiede beider Enzyme deutlich zu machen und die Vorzüge des Enzyms aus L. brevis aufzuzeigen, wurde die Alkohol-Dehydrogenase aus L. kefir analog zum L. brevis-Enzym bis zur Homogenität gereinigt und im Verlauf der Charakterisierung des L. brevis-Enzyms mitunter vergleichend geprüft (z.B. Temperaturstabilität, N-terminale Aminosäure-Sequenz). Anzucht von Lactobacillus kefir, die Enzymisolierung und Reinigung bis zum homogenen Enzym wurden wie in Beispiel 2A) - 2C) für L. brevis beschrieben durchgeführt. Tabelle 3 faßt die Reinigung des L. kefir-Enzyms zusammen.

Tabelle 3

| Reinigung der Alkohol-Dehydrogenase aus Lactobacillus kefir | | | |
|---|---|---|---|
| Reinigungsschritt | Aktivität [U/ml] | spez. Akt. [U/mg] | Ausbeute [%] |
| Rohextrakt | 57,5 | 9,15 | 100 |
| Phenylsepharose | 90,0 | 49 | 31 |
| Octylsepharose | 7.5 | 100 | 26 |
| Mono Q | 17.5 | 206 | 23 |
| 2′,5′-AMP-Seph. | 48.8 | 174 | 8.5 |

Beispiel 2 der Gewinnung und Reinigung der ADH aus L. brevis zeigt, daß dieses Enzym zum Teil ähnliche proteinchemische Eigenschaften wie das L. kefir-Enzym besitzt. Beide Enzyme benötigen beispielsweise $Mg^{2+}$-Ionen, um eine irreversible Schädigung zu vermeiden. Insbesondere bestehen jedoch die folgernden Unterschiede:

1. Die ADH-Enzyme aus L. brevis und L. kefir werden nach Bindung an ein Ionenaustauscher-Material (in diesem Fall MonoQ) durch signifikant unterschiedliche Salzkonzentrationen abgelöst. Diese unterschiedliche Bindungsstärke weist auf Unterschiede in der Aminosäure-Zusammensetzung bezüglich der geladenen Aminosäuren hin. Dies konnte durch die unten beschriebene Sequenzierung bestätigt werden.

2. Die Ausbeute an gereinigtem, enzymatisch aktivem Protein ist für das L. kefir-Enzym deutlich höher, was auf ein stabileres Enzym hinweist.

Beispiel 3:

Biochemische Charakterisierung der Alkohol-Dehydrogenase aus Lactobacillus brevis

A. pH-Stabilität

Die Abhängigkeit der Aktivität des Enzyms bei Lagerung in Puffern mit verschiedenen pH-Werten wurde im Bereich von pH 4 bis 11 untersucht. Es wurden je nach Pufferkapazität verschiedene Puffer im pH-Bereich von 4 bis 11 angesetzt und das homogene Enzym darin für 30 min inkubiert. Davon wurde 1 µl entnommen und zu dem normalen Testansatz von 970 µl Triethanolaminpuffer (100 mM; pH 7.0 mit 11 mM Acetophenon) und 20 µl NADPH (Endkonzentration 0.19 mM) hinzugegeben. Die Reaktion wurde über 1 min bei 30°C und 340 nm verfolgt. Dabei zeigten sich 2 Maxima der pH-Stabilität, ein kleineres bei pH 5.5 und ein großes bei pH 9.0. Die Stabilität ist in Tabelle 3 dargestellt.

Tabelle 4

| pH-Stabilität der Alkohol-Dehydrogenase | | |
|---|---|---|
| pH-Wert | Puffer | Aktivität [U/ml] |
| 4,0 | Na-acetat / Essigsäure | 6,86 |
| 4,5 | Na-acetat / Essigsäure | 6,22 |
| 5,0 | MES / NaOH | 7,04 |
| 5,5 | MES / NaOH | 8,73 |
| 6,0 | Triethanolamin / NaOH | 5,58 |
| 6,5 | Triethanolamin / NaOH | 5,80 |
| 7,0 | Triethanolamin / NaOH | 6,57 |
| 7,5 | Tris / HCl | 5,04 |
| 8,0 | Tris / HCl | 3,83 |
| 8,5 | Tris / HCl | 8,42 |
| 9,0 | Tris / HCl | 17,11 |
| 10,0 | Glycin / NaOH | 1,25 |
| 11,0 | Glycin / NaOH | 2,43 |

B. Temperaturstabilität

In analoger Weise wie unter A. beschrieben wurde die Temperaturstabilität für den Bereich von 25 bis 70°C bestimmt. Die homogene Enzymlösung wurde für 30 min den jeweiligen Temperaturen ausgesetzt und anschließend direkt bei 30°C mit dem obigen Testansatz gemessen. Die Alkoholdehydrogenase ist in dem Temperaturbereich zwischen 25 und 60°C für den angegebenen Zeitraum stabil (s. Tabelle 5), sie zeigt ein Maximum bei 40°C. Demgegenüber ist die ADH aus L. kefir nur bis 40°C stabil mit einem Maximum bei 37°C, danach sinkt die Aktivität rapide ab. Das Maximum dieses Enzym liegt bei 37°C.

Tabelle 5

| Temperaturstabilität der Alkohol-Dehydrogenase aus L. brevis | | | |
|---|---|---|---|
| L. brevis Temperatur [°C] | L. brevis Aktivität [U/ml] | L. kefir Temperatur [°C] | L. kefir Aktivität [U/ml] |
| 25 | 9,43 | 25 | 36.5 |
| 30 | 8,74 | 30 | 30.2 |
| 37 | 12,06 | 37 | 35.8 |
| 40 | 12,15 | 40 | 32 |
| 42 | 11,16 | 45 | 26.2 |
| 45 | 10,81 | 50 | 2.4 |
| 47 | 8,23 | 55 | 0 |
| 50 | 8,37 | | |
| 60 | 8,51 | | |
| 70 | 0,56 | | |

C. Temperaturoptimum

Zur Bestimmung der optimalen Testtemperatur wurde die Enzymaktivität zwischen 25 und 70°C gemessen. Der Testansatz entsprach den Standardkonzentrationen an Acetophenon und NADPH und wurde jeweils 5 min bei den angegebenen Temperaturen inkubiert. Das Enzym hat seine optimale Testtemperatur bei 50°C, wie aus der Tabelle 6 ersichtlich wird. Die Alkohol-Dehydrogenase aus L. kefir hat dagegen ein Optimum von 37°C und bei höheren Testtemperaturen als 45°C sinkt die Aktivität rapide ab (Tabelle 5).

Tabelle 6

| Temperatur-Optimum für die Aktivität der Alkohol-Dehydrogenase aus Lactobacillus brevis (im Vergleich zu der Alkohol-Dehydrogenase aus Lactobacillus kefir). | | | |
|---|---|---|---|
| L. brevis Temperatur [°C] | L. brevis Aktivität [U/ml] | L. kefir Temperatur [°C] | L. kefir Aktivität [U/ml] |
| 25 | 10.31 | 20 | 28.0 |
| 30 | 9.66 | 25 | 28.2 |
| 37 | 9.76 | 30 | 35.2 |
| 40 | 17.78 | 35 | 36.2 |
| 42 | 16.93 | 40 | 34.4 |
| 45 | 18.06 | 45 | 35.4 |
| 47 | 19.13 | 50 | 26.0 |
| 50 | 31.46 | 55 | 3.0 |
| 55 | 24.48 | 60 | 0 |
| 60 | 23.69 | | |
| 65 | 1.98 | | |
| 70 | 0.58 | | |

D. Substratspektrum der Alkohol-Dehydrogenase

Es wurden anstelle von Acetophenon eine Reihe von anderen Ketonen und Ketoestern und getestet, ob diese enzymkatalysiert reduziert werden können.

Folgender Testansatz wurde dafür verwendet:

970 µl Triethanolaminpuffer (50 mM; pH 7.0, mit 10 mM Ketoverbindung)
20 µl NADPH (0.19 mM im Test)
10 µl gereinigtes Enzym (s. Beispiel 2. nach Affinitätschromatographie, 1:10 verdünnt)

Teilweise wurden die Ketone auch in einer Konzentration von 1 mM getestet, um eine mögliche Substrathemmung bei 10 mM zu verifizieren. Es stellte sich jedoch heraus, daß eine solche Überschuß-Inhibierung bei einer Keton-Konzentration von 10 mM nicht auftrat. Die für die entsprechenden Substrate erhaltenen Aktivitäten sind in Tabelle 7 wiedergegeben.

Für Substrate wie p-Cl-Acetophenon, Methyl-1-naphtylketon und Methylcyclohexanon kann aufgrund von Literaturangaben ein direkter Vergleich zwischen der Alkohol-Dehydrogenase aus L. kefir und der aus L. brevis gezogen werden (Hummel, W. Appl. Microbiol. Biotechnol. (1990), 34, 15-19). Dieser Vergleich zeigt, daß die erfindungsgemäße ADH die genannten Verbindungen mit höherer Aktivität umsetzt; die Aktivitäten liegen ca. um 50 bis 100% höher als die für ADH aus L. kefir.

Tabelle 7

| Substratspektrum der Alkohol-Dehydrogenase aus L. brevis | | |
|---|---|---|
| Substrat | Aktivität [U/ml] | Relative Akt. [%] |
| Acetophenon | 3.26 | 100 |
| 4-Cl-Acetophenon | 6.63 | 203 |
| 3-Cl-Acetophenon | 4.80 | 147 |
| 2-Cl-Acetophenon | 0.33 | 10 |
| 4-Ethylacetophenon | 2.13 | 65 |
| 2-Methylcyclohexanon | 6.25 | 192 |
| 2-Oxo-4-phenylbuttersäureethylester | 4.60 | 141 |
| 2,4-Pentandion | 4.22 | 130 |
| Acetessigsäurebenzylester | 4.11 | 126 |
| Acetessigsäuremethylester | 3.81 | 117 |
| Acetessigsäureethylester | 3.54 | 109 |
| Benzylaceton | 3.24 | 99 |
| Methylpyruvat | 3.22 | 98 |
| Ethylpyruvat | 8.10 | 248 |
| 4-Cl-Acetessigsäureethylester | 3.04 | 93 |
| Laevulinsäureethylester | 2.99 | 92 |
| 4-Acetylbuttersäureethylester | 2.84 | 87 |
| 3-Oxo-n-valeriansäureethylester | 2.29 | 70 |
| 3-Oxo-n-valeriansäuremethylester | 2.26 | 69 |
| Ethylbutyrylacetat | 2.21 | 68 |
| Acetessigsäurebutylester | 2.07 | 64 |
| Isobutyrylessigsäureethylester | 1.93 | 59 |
| Methyl-1-naphthylketon | 1.21 | 37 |
| Benzoylessigsäureethylester | 1.14 | 35 |
| Ethyl-2-methylacetoacetat | 0.84 | 26 |
| 1,4-Butandiol-diglycidylether | 0.77 | 24 |
| Hydroxyaceton | 0.74 | 23 |
| Propiophenon | 0.55 | 17 |
| Trifluoracetessigsäureethylester | 0.50 | 15 |
| Cyclohexanon-2-carbonsäureethylester | 0.41 | 13 |
| Ethyl-2-oxo-4-phenylbutyrat | 0.40 | 12 |
| Benzylmethylketon | 0.36 | 11 |
| 4-Acetylbuttersäure | 0.32 | 10 |
| Benzaldehyd | 0.30 | 9 |

Tabelle 7 (fortgesetzt)

| Substratspektrum der Alkohol-Dehydrogenase aus L. brevis | | |
|---|---|---|
| Substrat | Aktivität [U/ml] | Relative Akt. [%] |
| Ketovaleriansäure | 0.27 | 8 |
| Cyclohexanon-2-essigsäureethylester | 0.14 | 4 |

Das Substratspektrum des erfindungsgemäßen Enzym ist somit ähnlich breit wie das des L. kefir-Enzyms. Beide Enzyme setzen Acetophenon-Derivate, 2- und 3-Oxoester, cyclische und offenkettige Ketone mit hoher Aktivität um und sind befähigt durch die Reduktion von Ketonen R-Alkohole zu bilden.

E. Molekulargewicht der Alkohol-Dehydrogenase aus Lactobacillus brevis

Gereinigte Alkohol-Dehydrogenase aus Lactobacillus brevis wurde auf eine Gelfiltrationssäule Superdex G-200 (Fa. Pharmacia, Freiburg) aufgetragen. Das Molekulargewicht wurde gemäß einer Eichgerade, die mit Proteinen bekannten Molekulargewichts erhalten wurde, bestimmt. Der Puffer bei der Gelfiltration hatte den für die Reinigung üblichen pH-Wert von 7.0 (100 mM TEA, pH 7.0, 0.2 M NaCl, 1 mM $MgCl_2$). Unter diesen Bedingungen wurde für die L. brevis-ADH ein Molekulargewicht von ca. 104 kD bestimmt, was dem der L. kefir-ADH entspricht. Zudem bestehen beide Enzyme aus vier Untereinheiten.

F. Bestimmung der N-terminalen Sequenz der Alkohol-Dehydrogenase

Die N-terminale Sequenz wurde mit einem Pulsed Liquid Sequencer Model 477A mit on-line HPLC Model 120A der Firma Applied Biosystems/U.S.A. bestimmt und mit der N-terminalen Sequenz der Alkohol-Dehydrogenase aus L. kefir verglichen.

S-N-R-L-D-G-K-V-A-I-V-T-G-G-T-L-G-I-G-L-A-I-A-T-K-F-V-E-E-G-A-K-V-M-I-T-T-R (SEQ. ID NO: 1)

Im Vergleich zur Sequenz aus L. kefir wurden bei den ersten 38 Aminosäuren (AS) folgende fünf Aminosäureaustausche verzeichnet:

Pos. 1 von Thr nach Ser (kein Unterschied im chemischen Verhalten der AS);
Pos. 2 von Asp nach Asn (saure, geladene AS wird gegen ungeladene ausgetauscht);
Pos. 5 von Lys nach Asp (basische wird gegen saure AS ausgetauscht);
Pos.24 von Asp nach Thr (saure gegen hydrophile AS ausgetauscht);
Pos.34 von Val nach Met (hydrophobe gegen schwefelhaltige AS ausgetauscht).

In vier von fünf Fällen liegen solche Aminosäureaustausche vor, die entgegengesetzte Polaritätseigenschaften an diesen Positionen im Enzym hervorrufen.

Beispiel 4:

Bestimmung von Aminosäure-Teilsequenzen nach Spaltung des Enzyms

Homogene Alkohol-Dehydrogenase aus Lactobacillus brevis wurde einer Spaltung mit Endoproteinase Lys-C Protease (Boehringer Mannheim, 476 986) unterworfen. Dafür mußte das Protein vorher denaturiert und carboxymetyliert werden. Die ADH-Probe wurde direkt in 60 µl Guanidinium-Puffer pH 8.5 aufgenommen [2 mg/ml] und bei RT 30 min inkubiert. Nach Ablauf der Inkubation wurde 1/10 Volumen einer 111 mM DTT-Lösung hinzugegeben und erneut für 30 min bei 37°C inkubiert. Zuletzt wurde 1/10 Volumen einer 360 mM Iodessigsäurelösung hinzugegeben und die Probe für 30 min im Dunkeln bei 37°C inkubiert. Die Reaktion wurde mit β-Mercaptoethanol abgestoppt. Nach Umpuffern auf einen Harnstoff-haltigen (2 M) Puffer wurde 1% (Endkonzentration) Triton X-100 (reduziert) und 4.6 µg Lys-C Protease (1/25 der Proteinmenge [w/w]) hinzugegeben und über Nacht bei 37°C inkubiert. Nach Ablauf der Zeit (mind. 16-18 h) wurde die Reaktion durch Zugabe von 1/10 Volumen 10% TFA abgestoppt. Von dem Ansatz wurden dreimal je x 150 µl und schließlich 1 x 100 µl in die HPLC eingespritzt. Jeder proteinhaltige Peak wurde getrennt gesammelt

und nach Vergleich der einzelnen Laufe gleiche Peaks zusammengenommen und in einer Vakuum-Zentrifuge eingeengt. Diese Proben (insgesamt 18) wurden dann direkt zum Sequenzieren verwendet.

Sequenzdaten (aufgeführt sind lediglich die wichtigsten Daten von Fraktionen mit eindeutiger, vollständiger Sequenzinformation):

Das Protein in Peakfraktion Nummer 3 hat folgende Sequenz V-M-I-T-G-R-H-S-D-V-G-E-K (SEQ. ID NO: 2) und knüpfte damit direkt an das N-terminale Ende an.

Die Sequenzen der Proteinfraktionen Nummer 5 (D-Y-D-V-R-V-N-T-V-H-P-G-Y-I-K, (SEQ. ID NO: 3)) und Nummer 6 konnten ebenfalls bestimmt werden.

Bei letzterer, F-A-T-G-S-E-F-V-V-D-G-G-Y-T-A-Q (SEQ. ID NO: 4) handelt es sich um das C-terminale Ende des Monomers der ADH aus *Laktobacillus brevis*.

Die Sequenzen der restlichen 12 Fraktionen sind hier nicht angeführt. Sämtliche Teilsequenzen konnten jedoch durch entsprechende Klonierungsexperimente bestätigt werden.

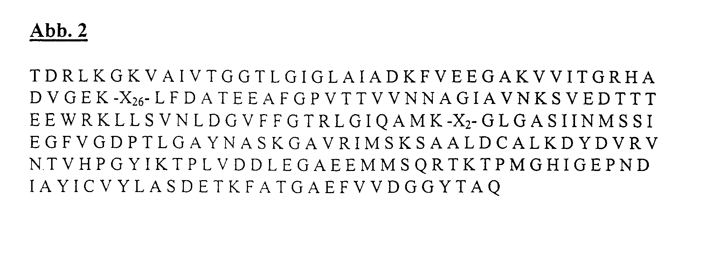

Ein entsprechendes Experiment zur Spaltung gereinigter ADH aus L. kefir mit der Protease LysC war erfolgreich. Bei Trennung der Peptide mittels HPLC ergab sich ein in hohem Maße ähnliches Spaltmuster wie bei der Spaltung des Enzyms aus L. brevis. Aus dieser Spaltung konnte nach Sequenzierung der Peptid-Spaltstücke nahezu die komplette Aminosäure-Sequenz des L. kefir Enzyms erhalten werden. Werden die nachgewiesenen Sequenzteile mit der Sequenz der ADH aus L. brevis verglichen (s. Abb. 2), zeigt sich, daß die Teilstücke der L. kefir-Sequenz im wesentlichen der L. brevis-Sequenz entsprechen, zwei entscheidende Teilstücke fehlen jedoch in der L. kefir-Sequenz. Diese Teilstücke sind in Abb. 2 gekennzeichnet durch $X_{26}$, d.h. ein Teilstück, welches 26 Aminosäuren umfassen müßte, um eine Abgleichung mit der L. brevis-Sequenz zu erreichen und mit $X_2$ für ein zwei Aminosäuren umfassendes Teilstück.

Ein Sequenzvergleich der ADHs aus L. brevis und L. kefir zeigt, daß - von den fehlenden Teilstücken $X_{26}$ und X2 abgesehen - 18 Aminosäuren, also ca. 10% ausgetauscht sind.

Beispiel 5:

Klonierung des Enzyms

A. Präparation genomischer DNA aus Lactobacillus brevis

Zellen von Lactobacillus brevis wurden mit 50 ml high TE-Puffer (25 mM Tris, 10 mM EDTA pH 8.0) gewaschen und bei 6000 rpm zentrifugiert. Das Pellet wurde in 10 ml TE (25 mM Tris, 10 mM EDTA pH 8.0) resuspendiert und unter Zugabe von 100 µl Lysozym (100 mg/ml) für 1.5 h bei 37°C inkubiert. Anschließend wurde über Nacht bei 50°C unter Zugabe von 340 µl 30% Natriumlaurylsarcosin, 100 µl Qiagen Protease (Qiagen, Hilden; 20 mg/ml) und 25 µl RNAse A (40 mg/ml) zu den lysierten Zellen das Zellprotein denaturiert und die RNA abgebaut. Im nächsten Schritt wurde aus dem Zellysat die DNA durch Phenolfällungen vom restlichen Zellinhalt abgetrennt. (Dazu wurde immer im Volumenverhältnis 1:1 zuerst reines Phenol (mit TE abgesättigt), dann Phenol/IAA/$CHCl_3$ (25:24:1) (IAA = Isoamylalkohol) und zuletzt IAA/$CHCl_3$ (24:1) auf die wäßrige Phase gegeben.) Die bereinigten, wäßrigen Phasen wurden durch sanftes Schwenken ca 5 min lang vermischt und dann durch Zentrifugation bei 20000 rpm getrennt. Die letzte wäßrige Oberphase wurde mit 0.0625 Volumen 8 M LiCl vermischt und dann mit zwei Volumenteilen kaltem Ethanol (100%) vorsichtig überschichtet. Die an der Grenzschicht ausfallende, genomische DNA wurde auf eine Pasteurpipette aufgewickelt und zweimal mit kaltem, 70%igem Ethanol gewaschen. Nach Trocknung in der Vakuumzentrifuge wurde die DNA in 2 ml TE (10 mM Tris, 1 mM EDTA) pH 8.0 aufgenommen. Zur Überprüfung des Reinheitsgrades der isolierten DNA wurde die Lösung 1:50 verdünnt und der Quotient 260:280 nm photometrisch als 2,0 bestimmt. Damit konnten Proteinverunreinigungen ausgeschlossen werden. Die Konzentration der DNA wurde aus dem Gel mit 70 ng/µl bestimmt, aus 3 g Zellen wurden 140 µg genomische DNA isoliert.

B. Oligonucleotide als 5'- und 3'-Primer für die PCR (Polymerase chain reaction)

Aufgrund enzymatischer Peptidspaltungen (s. Beispiel 4) konnten das N-terminale und C-terminale Ende der Proteinsequenz der ADH aus *L. brevis* bestimmt werden. Mit Hilfe dieser Informationen wurden Primer für die PCR synthetisiert. Dabei wurden bekannte Präferenzen für bestimmte Codons in Laktobacillen berücksichtigt. Vor jeden 5'primer wurde das Codon ATG (Met) als Startcodon vorgesetzt, was der Proteinsequenz fehlt, und hinter jeden 3'primer wurden 2 Stopcodons zur gesicherten Termination der Translation gesetzt. Die Primerkonstrukte sind im folgenden aufgelistet (In Klammern sind die variablen Nukleinsäuren aufgeführt, die alternativ zu der vor der Klammer stehenden Nukleinsäure eingebaut wurden, so daß der tatsächlich eingesetzte Primer ein Gemisch aus den sich ergebenden Kombinationen ist):

5'primer 5'LB:
5'ATG-TCA-AAC-CGT-TTA(G)-GAT-GGT(C)-AAG-GTT(A)-GCT(A)-ATT(C)-3'
(SEQ. ID NO: 5)

3'primer 3'LB:
5'CTA-CTA-TTG-A(T)GC-AGT-GTA-A(G)CC-A(G)CC-ATC-A(T)AC-A(T)AC-GAA-3'
(SEQ. ID NO: 6)

Die synthetisierten Primer wurden mit kaltem $NH_3$ von den Säulen eluiert. Diese $NH_3$-Lösung wurde für 1 h bei 70°C inkubiert und anschließend mit 1 ml Butanol ausgeschüttelt. Die Proben wurden bei 14000 rpm 2 min abzentrifugiert und der entstandene Überstand abdekantiert. Die Proben wurden zum Trocknen 5 min in die Vakuum-Zentrifuge gestellt und das Pellet in 500 µl $H_2O$ aufgenommen. Die Primer wurden in einer Konzentration von 100 pmol/µl eingesetzt.

C. PCR (Polymerase chain reaction) mit der genomischen DNA aus *L. brevis*

Im folgenden wurde die Templatekonzentration (genom. DNA) variiert, alle anderen Parameter wurden konstant gehalten. Als Annealingtemperatur wurde 52°C gewählt.

Tabelle 8

| PCR-Ansatz | | | | | | | |
|---|---|---|---|---|---|---|---|
| Ansatz | Template [ng] | 5'-Primer [pmol] | 3'-Primer [pmol] | dNTP [nM] | Puffer [10x] | Taq [U] | $H_2O$ [µl] |
| 1 | 700 | 100 | 100 | 1,6 | 10 µl | 2,5 | 61,5 |
| 2 | 70 | 100 | 100 | 1,6 | 10 µl | 2,5 | 70,5 |

Die jeweiligen Ansätze hatten ein Volumen von 100 µl, die molaren Angaben sind als Endkonzentrationen zu verstehen, das Template und die Taq DNA-Polymerase (Boehringer Mannheim) wurden in der in Tab. 8 aufgeführten Menge dem Gesamtansatz zugegeben. Der Puffer (PCR reaction buffer; Boehringer Mannheim) lag in 10facher Konzentration vor. Die PCR wurde mit 25 Cyclen über Nacht angesetzt. Es wurde ein PCR Gerät von Perkin Elmer mit beheizbarem Deckel verwendet.

Für die Analytik wurden 10% des Ansatzes (Volumenanteil) auf ein 1%iges Agarosegel aufgetragen und bei konstant 100 V elektrophoretisch aufgetrennt. Die PCR zeigt eine deutliche Amplifikation eines DNA-fragments von a 750 bp. Das Signal liegt zwischen den Markerbanden von 697 bp und 925 bp, wobei es näher an 697 bp reicht. Bei einem durch SDS-PAGE ermittelten Molekulargewichts des Monomers der ADH von 27 kD (245 AS) findet sich hier eine gute Übereinstimmung von Aminosäurelänge des ADH-Proteins und der Basenpaarlänge für das gesuchte Gen.

D. Reinigung der PCR-Produkte mit dem Qiaquick® PCR purification kit (Qiagen, Hilden)

Zu 100 µl PCR-Lösung wurden 500 µl Puffer PB pipettiert und direkt auf eine Quiaquick® Säule (ohne Äquilibrierung) aufgetragen. Die DNA wurde durch Zentrifugation (1 min, 14000 rpm) an die Säule gebunden, der Durchlauf wurde verworfen. Die Säule wurde mit 750 µl Puffer PE durch Zentrifugation (1 min, 14000 rpm) gewaschen und der Durchlauf verworfen. Die Säule wurde erneut kurz abzentrifugiert, um Reste des in dem Puffer PE enthaltenen Ethanols zu entfernen. Anschließend wurde die Säule unter Zugabe von 50 µl 2 mM Tris/HCl pH 8.0 durch Zentrifugation (1min, 14000 rpm) eluiert. Von diesem Eluat wurde zur Kontrolle je 1 µl auf ein analytisches 0.8%iges Agarosegel aufgetragen und unter 60 V konstant elektrophoretisch aufgetrennt. Die Konzentrationsbestimmung ergab 70ng/µl für PCR3/1 und 90ng/µl für PCR3/2. PCR3/2 wurde weiterverarbeitet, PCR3/1 wurde bei -20°C aufbewahrt. PCR3/2 wurde auf 16 µl in der Speed vac eingeengt und direkt für die Blunting/kinasing Reaktion des sureclone kits eingesetzt (16 µl PCR, 1 µl Klenow Fragment, 2 µl 10x Puffer, 1 µl Polynucleotidkinase; 30 min bei 37°C inkubieren). Das Reaktionsgemisch wurde direkt auf ein präparatives 0.8% Agarosegel (2 h, 80 V konstant) aufgetragen. Die bei 750 bp sichtbare Bande wurde aus dem Gel ausgeschnitten und über Jetsorb® (Genomed, Bad Oeynhausen) aus dem Gel isoliert. Die Plasmid DNA wurde aus dem Jetsorbmaterial mit 50 µl 2 mM Tris/HCl pH 8.0 eluiert. Diese Lösung wurde mit Hilfe einer

Vakuumzentrifuge auf 9 µl eingeengt. Davon wurde 1 µl für ein analytisches Agarosegel verwendet.

Aus der Konzentration des PCR Fragments im Gel (150 ng/µl) ergibt sich eine Gesamtkonzentration an DNA von 1.2 µg. Diese Probe wurde in den Ligaseansatz des sureclone kits eingesetzt.

E. Jetsorb® DNA Isolierung aus dem Gel

Pro 100mg Gelmaterial wurden 300 µl Puffer A1 und 10 µl der Jetsorbsuspension vermischt und bei 50°C für 15 min inkubiert. Dabei wurde von Zeit zu Zeit die Lösung neu gemischt, um eine komplette Bindung an das Jetsorbmaterial zu gewährleisten. Anschließend wurde die Suspension 30 sec bei 11000 rpm abzentrifugiert, der Überstand verworfen, das Pellet in 300 µl Puffer A2 resuspendiert und erneut bei 11000 rpm abzentrifugiert. Dieser Schritt wurde komplett wiederholt und anschließend der Überstand sauber abpipettiert und das Pellet bei 50°C 5 min getrocknet. Dann wurde das Pellet in 30 µl 2 mM Tris/HCl ph 8.0 resuspendiert und 5 min bei 50°C inkubiert. Nach erfolgter Zentrifugation (11000 rpm, 30 sec) wurde der Überstand gesammelt und das Pellet erneut in 20 µl 2 mM Tris/HCl pH 8.0 resuspendiert und wie oben weiter behandelt. Die resultierenden Überstände wurden vereinigt und in der Speed vac auf 18 µl eingeengt. Davon wurde 1 µl auf eine analytisches Agarosegel aufgetragen. Die Konzentrationsbestimmung über dieses Gel ergab eine DNA Konzentration von 10 ng/µl, also eine Gesamtkonzentration von 160 ng.

F. Ligation mit dem Sureclone Kit (Fa. Pharmacia)

Eingesetzt wurden 7 µl PCR (1 µg), 1 µl pUC18 Vektor (50ng), 1 µl DTT, 10 µl zweifachen Ligasepuffer. 1 µl Ligase. Von diesem Ansatz wurden 3 µl entnommen und in einem neuen Ligaseansatz eingesetzt, um die PCR-Fragmentkonzentration zu erniedrigen. Ein zweiter Ansatz entspricht bis auf die PCR-DNA Konzentration (157 ng, d.h. ein Verhältnis von 1:3 Vektor : PCR) in der Zusammensetzung dem ersten Ansatz (Verhältnis von 1:20 Vektor: PCR). Beide Ansätze wurden für 1,5 h bei 16°C inkubiert und anschließend je 100 µl kompetente Zellen (Escherichia coli XL 1 Blue) damit transformiert. Von Ligaseansatz 1 wurden 1 µl und von Ligaseansatz 2 wurden 4 µl zu den kompetenten Zellen pipettiert. Von der Zellsuspension wurden je 300 µl auf $LB_{amp}$-Agarplatten ausplattiert (LB=Caseinpepton/Hefeextrakt; pH 7,5).

Gewachsene Kolonien wurden abgeimpft und in 4 ml Flüssigkultur ($LB_{amp}$-Medium) über Nacht bei 37°C kultiviert. Von dieser Zellsuspension wurden je 2 ml zur Plasmidpräparation (entsprechend dem Qiagen miniprep Protokoll (Qiagen, Hilden); s.u.) eingesetzt. Die Plasmid DNA wurde aus der zellfreien Lösung direkt mit 0,7 Volumenteilen Isopropanol gefällt, mit 70% kalten Ethanol gewaschen, in der Vakuumzentrifuge getrocknet und in 10 µl 2 mM Tris/HCl-Puffer (pH 8.0) aufgenommen. Von dieser Plasmidpreparation wurde ein Restriktionsverdau mit Eco R1 und Hind III angesetzt (1 U/µl Enzym, 6 µl Plasmid DNA. 37°C, 1 h). Der komplette Verdau wurde auf ein 0.8%iges Agarosegel aufgetragen. 2 Std. bei konstant 80 V elektrophoretisch aufgetrennt (Nachweis des 750 kb-Inserts) und die Plasmide daraufhin gegebenenfalls für die Sequenzierung eingesetzt.

G. Plasmid-Präparation (Qiagen Miniprep)

4 ml der Flüssigkultur wurden bei 5000 rpm 5 min abzentrifugiert und das Pellet in 300 µl Puffer P1 resuspendiert. Zu dieser Suspension wurden 300 µl Puffer P2 gegeben und die Zellsuspension bei Raumtemperatur für 5 min inkubiert. Anschließend wurde den lysierten Zellen 300 µl kalter Puffer P3 hinzupipettiert und für 10 min auf Eis unter mehrmaligem Schwenken inkubiert, um Nukleinsäuren und Proteine zu denaturieren. Die denaturierten Bestandteile wurden durch Zentrifugation (11000 rpm, 15 min) abgetrennt; der Überstand direkt auf neue, mit 1 ml QBT Puffer (Qiagen, Hilden) äquilibrierte Qiagen 20 Tip Säulen (Qiagen, Hilden) gegeben. Die Säulen wurden mit 4 x 1 ml Puffer QC gewaschen und anschließend die Plasmid DNA mit 0.8 ml Puffer QF von den Säulen eluiert. Aus dieser Lösung wurde die DNA mit 0.7 Vol Isopropanol nach 30 min Zentrifugation bei 14000 rpm gefällt. Das resultierende Pellet wurde 2 x 10 min mit 70% Ethanol während der Zentrifugation bei 14000 rpm gewaschen und dann in der Vakuumzentrifuge getrocknet. Die DNA wurde in 10 µl 2 mM Tris/HCl pH 8.0 aufgenommen und beispielsweise für Analysen mittels Restriktionsenzymen eingesetzt.

H. DNA Sequenzierung der Plasmide

a. Fertigen des Sequenzgels

Die Sequenzierung wurde mit dem autoread laser fluorescent Sequencer (ALF) der Firma Pharmacia durchgeführt, dementsprechend wurde der autoread Sequencing kit (Pharmacia) für die Sequencierungsreaktion verwendet. Das Gel wies standardmäßig eine Dicke von 0.5 mm auf. An der Stelle des Laserdurchtritts wurde ein entsprechendes Glasplättchen (Dicke 0.35 mm) eingesetzt. Sequenzgels: 21 g Harnstoff (ultra pure), 12 g $H_2O$ (Millipore), 7.5 ml Acrylamid (Roth, zur DNA Sequenzierung), 200 µl APS, 45 µl TEMED, 6 ml 10-fach TBE. Als Laufpuffer diente 0.6 x TBE (Tris/Borat/EDTA-Puffer entsprechend Pharmacia-Angaben).

b. Sequenzierungsreaktion mit dem AutoRead™ Sequencing kit

Von den Plasmiden wurden je 5 µl (4 µg) DNA pro sequencing primer eingesetzt.

Es wurden 5 µl Plasmid DNA, 5 µl $H_2O$, 2 µl Primer (universal oder reversal), 1,5 µl 1 M NaOH zusammenpipettiert und für 5 min bei 65°C zur DNA Denaturierung inkubiert. Dann wurden die Proben direkt nach 37°C überführt und die Reaktion mit 1,5 µl 1 M HCl neutralisiert. Es wurden 2 µl Annealingpuffer hinzugegeben, kurz abzentrifugiert (15 sec, 14000 rpm) und 10 min bei 37°C, anschließend weitere 10 min bei Raumtemperatur inkubiert. Nach erfolgter Zentrifugation wurden 1 µl Extensionpuffer und 3,5 µl Dimethylsulfoxid mit dem Ansatz vermischt und pro Ansatz 2 µl der mit Enzymdilutionspuffer verdünnten T7-Polymerase pipettiert. 5,2 µl dieser Lösung wurden direkt in eine bei 37°C vorinkubierte Platte pipettiert, die pro Plasmid 4 Spuren mit 3 µl des jeweiligen NTP-Mixes (A, C, G, T, in der Reihenfolge) enthielt. Nach 5 min Inkubation bei 37°C wurde die Sequenzierungsreaktion nach Sanger (Sanger, F., Nickler, S., Coulson, A.R. (1977) Proc. Natl. Acad. Sci. USA 74, 5463-5467) durch Zugabe von 6 µl Stopmix abgestoppt, und das Reaktionsgemisch für 2-3 min bei 90°C erhitzt, danach direkt auf Eis gekühlt, um ein Renaturieren der Stränge zu verhindern. Dann wurden direkt 6 µl dieses Gemisches auf das Sequenzgel aufgegeben und der Sequencer gestartet. Von den ausgewählten Plasmiden wies nur das Plasmid 3/1 die richtige DNA-Sequenz auf; diese Daten können nun in die entsprechende Proteinsequenz übersetzt werden (Abbildung 1). Der universal Primer zeigt das C-terminale Ende der Sequenz und der reversal Primer das N-terminale Ende (5'codierender Strang). Daher ist das Insert in der richtigen Orientierung plaziert. Die Sequenz auf Grund der DNA-Sequenzierung zeigt völlige Übereinstimmung mit den durch Proteinsequenzierung erhaltenen Sequenzdaten.

Beispiel 6:

A. Konstruktion des pADH Expressionsplasmides

Zur Expression der Alkoholdehydrogenase wurde das Strukturgen in den pKK177-Expressionsvektor so kloniert, daß das Strukturgen in der richtigen Orientierung unter Kontrolle eines geeigneten IPTG-induzierbaren Promoters, bevorzugt dem Tac-Promotor insertiert ist. Dafür wurde das Strukturgen für die Alkoholdehydrogenase mittels EcoRI und HindIII aus dem pUC-Vektor mit dem ADH-Gen ausgeschnitten, der Restriktionsansatz durch Agarosegelelektrophorese aufgetrennt und das ca. 750 Bp große Fragment aus dem Agarosegel isoliert. Gleichzeitig wurde das Expressionsplasmide pKK177 und mit EcoRI und HindIII geschnitten, der Restriktionsansatz durch Agarosegelelektrophorese aufgetrennt und das ca. 2,8 kBp Vektorfragment aus dem Agarosegel isoliert. Die so gewonnenen Fragmente wurden wie beschrieben miteinander ligiert. Das neu entstandene Plasmid wurde pADH-1 genannt. Die ordnungsgemäße Insertion des Gens wurde mittels Restriktionsanalyse und Sequenzierung nachgeprüft.

B. Transformation des Expressionsplasmides pADH-1 in verschiedene Escherichia coli Expressionsstämme

Kompetente Zellen verschiedener Escherichia coli Stämme, bevorzugt E. coli B HB101 und E. coli K12 NM522, wurden entsprechend der Methode nach Hanahan (J. Mol. Biol. 166 (1983) pp. 557) hergestellt. 200 µl derart hergestellter Zellen wurden mit 20 ng isolierten pADH-1 Plasmid-DNA versetzt. Nach 30 min Inkubation auf Eis erfolgte ein Hitzeschock (90 sec bei 42°C). Anschließend wurden die Zellen in 1 ml LB-Medium überführt und zur phäntotypischen Expression 1 Stunde bei 37°C inkubiert. Aliquote dieses Transformationsansatzes wurden auf LP-Platten mit Ampicillin als Selektionsmarker ausplattiert und 15 Stunden bei 37°C inkubiert.

C. Expression der Alkoholdehydrogenase aus Lactobacillus brevis in Escherichia coli

Transformanden, die das Expressionsplasmid pADH-1 aufgenommen haben, wurden gepickt und einzeln in 3 ml LB-Flüssigmedium mit Ampicillin überimpft und bei 37°C im Roller inkubiert. Bei einer optischen Dichte (Meßwellenlänge 550 nm) von 0,5 wurden die Zellen mit 1 mM IPTG induziert. Als Kontrolle wurde jeweils ein Klon mit Expressionsplasmid, aber ohne Induktion und der jeweilige E. coli WT ohne Expressionsplasmid, aber IPTG induziert, mitgeführt. 4 Stunden nach der Induktion und nach Wachstum über Nacht wurde jedem Röhrchen ein Aliquot entnommen, das einer optischen Dichte von $OD_{550}$ = 5 entsprach. Die Zellen wurden abzentrifugiert (10 min bei 6000 upm, 4°C), in 300 µl TE-Puffer (50 mM Tris-HCl, 50 mM EDTA, pH 8,0) aufgenommen und mittels Ultraschall aufgeschlossen (Branson Sonifire 450/2 x 30 sec bei Stufe 2 auf Eis). Durch Zentrifugation (10 min, 14000 upm) wurden die löslichen Proteine von den unlöslichen Proteinen und Zellwandbestandteilen getrennt. An dieser Stelle wurden ein Aliquot zur Durchführung des Aktivitätstestes entnommen. Der restliche Überstand wurde zur Vorbereitung für die SDS-Polyacrylamidgelektrophorese mit 50 µL 5x Auftragspuffer (60 mM Tris-HCl pH 6,8, 1,2% SDS, 10% Glycerin, 0,7 M 2-Mercaptoethanol, 0,05% Bromphenolblau) versetzt. Das Proteinpellet (unlösliche Proteine und Zellwandbestandteile) wurde wiederum in 300 µl TE-Puffer (50 mM Tris-HCl, 50 mM EDTA, pH 8,0) aufgenommen und durch 5 Impulse Ultraschall (Branson Sonifire 450) resuspendiert. Auch diese Proben wurden it dem entsprechenden Volumen 5x Auftragspuffer versetzt. Zuletzt wurden alle Proben 5 min auf 95°C erhitzt, um die Proteine vollständig zu denaturieren.

D. SDS-Polyacrylamidgelelektrophorese (SDS-PAGE)

Die SDS-PAGE wurde nach der Methode von U.K. Laemmli (1970, Nature 227, pp. 680-685). Die löslichen und unlösliche Proteinfraktionen wurden separat in einem 15%igen Trenngel aufgetrennt und die Proteinbanden anschließend mit Coomassie-Färbelösung (0,2% Coomassie, 30% Ethanol, 10% Essigsäure) angefärbt.

Die Alkoholdehydrogenase aus Lactobacillus brevis von den verwendeten E. coli-Stämmen wurde ausschließlich als lösliches Protein exprimiert. Dies konnte anhand einer zusätzlichen Proteinbande mit der zuvor bereits ermittelten Größe im SDS-PAGE von ~ 27 kDA ausschließlichen bei den löslichen Proteinfraktionen gezeigt werden. Diese zusätzliche Proteinbande war bei den Kontrollen (nichtinduzierter, plasmidhaltiger Klon und induzierter, nicht plasmidhaltiger Klon) sowie in den Proben mit den unlöslichen Proteinfraktion nicht zu sehen.

Ein mit dem Rohextrakt durchgeführter Aktivitätstest zeigte auch, daß die Alkoholdehydrogenase nicht nur in löslicher Form, sondern zudem in aktiver Form exprimiert wurde.

Beispiel 7:

Enzymkatalysierte Herstellung von R-Phenylethanol

Für die enzymkatalysierte Synthese von (R)-Phenylethanol aus Acetophenon wurden gereingtes Enzym (Beispiel 2C) und das erforderliche Coenzym (NADP) eingesetzt. Das oxidierte Coenzym wurde durch gleichzeitig anwesendes Isopropanol kontinuierlich regeneriert, so daß die Reaktion nur katalytische Mengen an Coenzym erfordert. Der Ansatz enthielt im einzelnen (in Klammern aufgeführt sind die Endkonzentrationen der Komponenten im Test): 20 µl NADP (0,05 mM), 7,7 µl Isopropanol (0,1 M), 0,6 µl Acetophenon (5 mM), 921,7 µl Triethanolamin-Puffer (50 mM; pH 7,0, mit 1 mM $MgCl_2$) und 50 µl Alkohol Dehydrogenase aus Lactobacillus brevis (4,5 Units). Dieser Ansatz mit einem Gesamtvolumen von 1 ml wurde für 24 Std. bei 30°C inkubiert, dann wurde eine Probe gaschromatographisch analysiert. Dazu wurden 100 µl entnommen, mit 100 µl Chloroform ausgeschüttelt, die Phasen durch Zentrifugation getrennt und aus der Unterphase (Chloroform) ein Aliquot für die GC entnommen.

Trennbedingungen für die Trennung von (R)- und (S)-Phenylethanol am Gaschromatographen: Stationäre Phase: Lipodex E, CD-Säule (Macherey und Nagel, Düren), Mobile Phase: Helium, Injektionsvolumen: 1 µl, Detektion: FID, Temperatur: 110°C. Unter diesen Bedingungen wird das Substrat Acetophenon bei 10,5 min eluiert, (S)-Phenylethanol bei 13,8 min und (R)-Phenylethanol bei 14,2 min. Die Retentionswerte für (S)- und (R)-Phenylethanol können mit einem kommerziell erhältlichen, racemischen Gemisch (Fa. Fluka, Buchs, Schweiz) erhalten werden. Nach 24 Std. sind mehr als 95% des Substrats umgesetzt worden, das GC-Chromatogramm zeigt folgende Werte: Acetophenon (Retentionszeit 10,5 min): area = 9.800; (S)-Phenylethanol (Retentionszeit 13,8 min): area = < 500; (R)-Phenylethanol (Retentionszeit 14,2 min): area = 240.000.

Beispiel 8:

Herstellung von Alkoholen durch Reduktion von Ketonen mit ganzen Zellen von Lactobacillus brevis

Eine Reduktion von Ketonen zu chiralen Alkoholen ist darüber hinaus mit ganzen Zellen von L. brevis möglich. Auf einen Zusatz von NADP kann dabei verzichtet werden, da in diesem Fall intrazellulär vorhandenes Coenzym ausgenutzt wird. Die Coenzym-Regenerierung kann durch Isopropanol oder durch metabolisierbare Substrate wie Glucose erreicht.

Testansatz: 50 mg Zellen von L. brevis (Feuchtgewicht nach Abzentrifugieren) werden in 860 µl Triethanolamin-Puffer (50 mM; pH 7,50; Zusatz von 1 mM $MgCl^{2+}$) suspendiert, der 0,1 M Glucose und 10 mM Acetophenon enthält. Proben werden mittels Gaschromatographie analysiert. Nach 2 h Inkubation bei 30 °C ist kein Acetophenon mehr nachweisbar, dafür hat sich eine entsprechende Menge an Phenylethanol gebildet. Da die Quantifizierung mittels GC-Trennung an einer chiralen stationären Phase durchgeführt wurde, konnte gleichzeitig gezeigt werden, daß das gebildete Phenylethanol von hoher optischer Reinheit ist, es konnte kein quantifizierbarer (S)-Alkohol nachgewiesen werden. Die optische Reinheit ist deutlich >95% für den (R)-Alkohol. Das zeigt, daß Zellen von L. brevis anscheinend keine weitere Alkohol-Dehydrogenase enthalten, die die optische Reinheit von gebildeten Alkoholen stören könnte, so daß chirale Alkohole auch auf diesem Weg unter Verwendung ganzer Zellen hergestellt werden können.

Legende zu Abbildung 1:

DNA- und Proteinsequenz der rekombinanten ADH aus *L. brevis* in *E. coli* (obere Zeile jeweils die Nukleinsäuresequenz (SEQ. ID NO: 7), darunter die dem Triplettcode entsprechende Aminosäure-Sequenz (im Einbuchstaben-Code, SEQ. ID NO: 8)). Die Sequenz beginnt mit Methionin, das über einen Start-Codon eingefügt worden ist. Dieses Methionin findet man nicht bei der entsprechenden Aminosäure-Sequenz, die durch Sequenzierung des reinen Proteins erhalten wurde (N-terminales Ende; s. Beispiel 3F und Beispiel 4).

Legende zu Abbildung 2:

Aminosäure-Sequenz (SEQ. ID NO. 9) der Alkohol-Dehydrogenase aus Lactobacillus kefir, erhalten durch Sequenzierung von Peptiden nach Spaltung des Enzyms durch LysC-Protease.

**Patentansprüche**

1. Stabiles mikrobielles Enzym mit Alkohol-Dehydrogenase-Aktivität, **dadurch gekennzeichnet, daß** es nach 30 Minuten bei 50° bis 60°C eine stabile Enzymaktivität aufweist, und in Aminosäureposition 1, 2, 5, 24 und/oder 34 entsprechend einen Serin-, Asparagin-, Asparaginsäure-, Threoninund/oder Methioninrest aufweist und bis zu einer - spezifischen Aktivität von 400 U/mg bis zur Homogenität des Enzyms reinigbar ist.

2. Enzym nach Anspruch 1, **dadurch gekennzeichnet, daß** es ein Stabilitätsmaximum bei 40°C und pH 9,0 aufweist.

3. Enzym nach Anspruch 1 oder 2, **dadurch gekennzeichnet, daß** das Enzym ein Stabilitätsmaximum bei pH 5,5 aufweist.

4. Enzym nach einem der vorangegangenen Ansprüche, **dadurch gekennzeichnet, daß** das Aktivitätsmaximum des Enzyms bei 50°C liegt.

5. Enzym nach einem der vorangegangenen Ansprüche, **dadurch gekennzeichnet, daß** es ein Molekulargewicht von 104 kDa (Gelfiltration) aufweist.

6. Enzym nach einem der vorangegangenen Ansprüche, **dadurch gekennzeichnet, daß** das Enzym aus Stämmen der Gattung Lactobacillus erhältlich ist.

7. Enzym nach einem der vorangegangenen Ansprüche erhältlich aus Lactobacillus brevis (DSM 20054).

8. Stabile Alkohol-Dehydrogenase, **dadurch gekennzeichnet, daß** das Enzym im wesentlichen eine Aminosäuresequenz gemäß SEQ.ID NO:8 aufweist.

9. Stabile Alkohol-Dehydrogenase für die eine DNA-Sequenz gemäß SEQ.ID NO:7 bzw. entsprechende Analoga codieren.

10. Verfahren zur Gewinnung des Enzyms nach einem der Ansprüche 1 bis 5, **dadurch gekennzeichnet, daß** eine gegebenenfalls mit einem für ADH codierenden Gen transformierte Mikroorganismusprobe aufgeschlossen wird und anschließend einer hydrophoben Interaktionschromatographie, einer Anionenaustausch- und Affinitätschromatographie in geeigneten, Magnesiumionen enthaltenen Puffersystemen unterworfen wird.

11. Verfahren nach Anspruch 8, **dadurch gekennzeichnet, daß** es sich bei dem Mikroorganismus um Lactobacillus brevis (DSM 20054) handelt.

12. Verfahren zur enantioselektiven Reduktion von organischen Ketoverbindungen, **dadurch gekennzeichnet, daß** eine Verbindung der allgemeinen Formel (I)

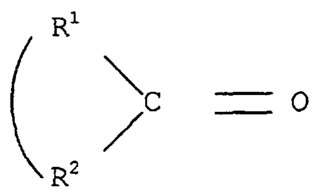

(I)

wobei $R^1$ oder $R^2$, verschieden oder identisch, Wasserstoff, zusammen oder einzeln, ein Alkyl-, Alkenyl-, Aryl- oder Arylenylrest, jeweils verzweigt oder geradkettig, bestehend aus 1 bis 20 C-Atomen, die durch ein oder mehrere Halogenatome, Nitro-, Hydroxyl- oder Alkoxyreste, wobei die Alkoxyreste aus 1 bis 20 C-Atomen aufweisen, eine gegebenenfalls substituierte Cl-C10-Alkylengruppe, die durch gesättigte, ungesättigte oder aromatische Stickstoff-, Sauerstoff-, oder Schwefelheterocyclen substituiert sind, oder ein gegebenenfalls substituierter polykondensierter gesättigter und/oder aromatischer Rest sein können,
**dadurch gekennzeichnet, daß** eine Ketoverbindung bzw. ein entsprechendes Gemisch in Gegenwart einer Alkohol-Dehydrogenase gemäß einem der Ansprüche 1 bis 9 bzw. das Enzym enthaltenen Zellen zwischen ca. 20° und 60°C über einen Zeitraum von ca. 15 Minuten bis 3 Stunden inkubiert und die entsprechende R-Hydroxyverbindung isoliert wird.

**13.** Verfahren zur enantioselektiven Synthese von organischen Hydroxyverbindungen gemäß der allgemeinen Formel (II)

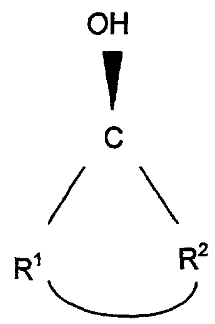

(II)

wobei $R^1$ oder $R^2$, verschieden oder identisch, Wasserstoff, zusammen oder einzeln, ein Alkyl-, Alkenyl-, Aryl- oder Arylenylrest, jeweils verzweigt oder geradkettig, bestehend aus 1 bis 20 C-Atomen, die durch ein oder mehrere Halogenatome, Nitro-, Hydroxyl- oder Alkoxyreste, wobei die Alkoxyreste aus 1 bis 20 C-Atomen aufweisen, eine gegebenenfalls substituierte C1-C10-Alkylengruppe, die durch gesättigte, ungesättigte oder aromatische Stickstoff-, Sauerstoff- oder Schwefelheterocyclen substituiert sind, oder ein gegebenenfalls substituierter polykondensierter gesättigter und/oder aromatischer Rest sein können,
**dadurch gekennzeichnet, daß** ein racemisches Gemisch der organischen Hydroxyverbindung mit einer Alkohol-Dehydrogenase gemäß der Ansprüche 1 bis 9 bzw. das Enzym enthaltenen Zellen zwischen ca. 20° und 60°C über einen Zeitraum von ca. 15 Minuten bis 3 Stunden inkubiert und die entsprechende S-Hydroxyverbindung isoliert wird.

**14.** Verfahren nach Anspruch 10 oder 11, **dadurch gekennzeichnet, daß** Magnesiumionen zugegen sind und der pH-Wert zwischen 6 und 9 liegt.

**15.** Verfahren zur rekombinanten Herstellung einer stabilen Alkohol-Dehydrogenase, **dadurch gekennzeichnet, daß** die Nukleotidsequenz gemäß SEQ. ID NO: 7 in einer eukaryotischen oder prokaryotischen Zelle exprimiert wird.

**16.** Isoliertes DNA-Fragment, welches für eine stabile Alkohol-Dehydrogenase codiert, **dadurch gekennzeichnet,**

**daß** es eine Nukleinsäuresequenz gemäß SEQ. ID NO: 7 bzw. eine damit hybridisierende Sequenz enthält.

**Claims**

1. A stable microbial enzyme with alcohol dehydrogenase activity, **characterised in that** it has stable enzyme activity after 30 minutes at 50 ºC to 60 ºC, and has a serine, asparagine. aspartic acid, threonine and/or methionine radical in amino acid position 1, 2, 5, 24 and/or 34 accordingly, and can be purified to homogeneity of the enzyme up to a specific activity of 400 U/mg.

2. An enzyme according to claim 1, **characterised in that** it has a stability maximum at 40 ºC and pH 9.0.

3. An enzyme according to claim 1 or 2, **characterised in that** the enzyme has a stability maximum at pH 5.5.

4. An enzyme according to one of the preceding claims, **characterised in that** the activity maximum of the enzyme is at 50 ºC.

5. An enzyme according to one of the preceding claims, **characterised in that** it has a molecular weight of 104 kDa (gel filtration).

6. An enzyme according to one of the preceding claims, **characterised in that** the enzyme may be obtained from strains of the Lactobacillus genus.

7. An enzyme according to one of the preceding claims which may be obtained from Lactobacillus brevis (DSM 20054).

8. Stable alcohol dehydrogenase, **characterised in that** the enzyme has an amino acid sequence substantially in accordance with SEQ ID NO: 8.

9. Stable alcohol dehydrogenase for which a DNA sequence in accordance with SEQ ID NO: 7 or corresponding analogues code.

10. A process for the preparation of the enzyme according to one of claims 1 to 5, **characterised in that** a microorganism sample optionally transformed with a gene coding for ADH is digested and then undergoes hydrophobic interaction chromatography. anion exchange and affinity chromatography in suitable buffer systems containing magnesium ions.

11. A process according to claim 8, **characterised in that** the microorganism is Lactobacillus brevis (DSM 20054).

12. A process for the enantioselective reduction of organic keto compounds, **characterised in that** a compound corresponding to the general formula (I)

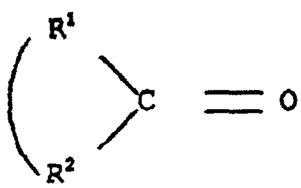

(I)

wherein $R^1$ or $R^2$, identical or different, may be hydrogen, together or individually, an alkyl, alkenyl, aryl or arylenyl radical, in each case branched or straight-chain, composed of 1 to 20 carbon atoms, which are substituted by one or more halogen atoms, nitro. hydroxyl or alkoxy radicals, the alkoxy radicals having 1 to 20 carbon atoms, an optionally substituted C1-C10 alkylene group. which are substituted by saturated, unsaturated or aromatic nitrogen, oxygen or sulfur heterocycles. or an optionally substituted polycondensed saturated and/or aromatic radical. **char-**

acterised in that a keto compound or a corresponding mixture is incubated in the presence of an alcohol dehydrogenase according to one of claims 1 to 9 or cells containing the enzyme at a temperature from 20 ºC to 60 ºC over a period from about 15 minutes to 3 hours and the corresponding R-hydroxy compound is isolated.

**13.** A process for the enantioselective synthesis of organic hydroxy compounds according to the general formula (II)

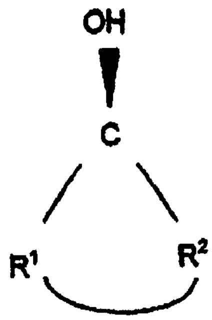

(II)

wherein $R^1$ or $R^2$, identical or different, may be hydrogen, together or individually, an alkyl, alkenyl, aryl or arylenyl radical, in each case branched or straight-chain, composed of 1 to 20 carbon atoms, which are substituted by one or more halogen atoms, nitro, hydroxyl or alkoxy radicals, the alkoxy radicals having 1 to 20 carbon atoms, an optionally substituted C1 I C10 alkylene group, which are substituted by saturated, unsaturated or aromatic nitrogen, oxygen or sulfur heterocycles, or an optionally substituted polycondensed saturated and/or aromatic radical, **characterised in that** a racemic mixture of the organic hydroxy compound is incubated with an alcohol dehydrogenase according to claims 1 to 9 or cells containing the enzyme at a temperature from about 20 ºC to 60 ºC over a period from about 15 minutes to 3 hours and the corresponding S-hydroxy compound is isolated.

**14.** A process according to claim 10 or 11. **characterised in that** magnesium ions are present and the pH is from 6 to 9.

**15.** A process for the recombinant preparation of a stable alcohol dehydrogenase, **characterised in that** the nucleotide sequence according to SEQ ID NO: 7 is expressed in a eukaryotic or prokaryotic cell.

**16.** An isolated DNA fragment, which codes for a stable alcohol dehydrogenase, **characterised in that** it contains a nucleic acid sequence according to SEQ ID NO: 7 or a sequence hybridising therewith.

**Revendications**

**1.** Enzyme microbienne stable dotée d'une activité d'alcool déshydrogénase, **caractérisée en ce qu'**elle présente une activité enzymatique stable au bout de 30 minutes à une température située entre 50°C et 60°C et présente un radical sérine, asparagine, acide asparaginique, thréonine et/ou méthionine de manière correspondante en position d'acide aminé 1, 2, 5, 24 et/ou 34 et est purifiable, jusqu'à homogénéité de l'enzyme, jusqu'à une activité spécifique de 400 U/mg.

**2.** Enzyme selon la revendication 1, **caractérisée en ce qu'**elle présente un maximum de stabilité à 40°C et à pH 9,0.

**3.** Enzyme selon la revendication 1 ou 2, **caractérisée en ce que** l'enzyme présente un maximum de stabilité à pH 5,5.

**4.** Enzyme selon l'une des revendications précédentes, **caractérisée en ce que** le maximum de l'activité de l'enzyme se situe à 50°C.

**5.** Enzyme selon l'une des revendications précédentes, **caractérisée en ce qu'**elle présente un poids moléculaire de 104 kDa (filtration sur gel).

6. Enzyme selon l'une des revendications précédentes, **caractérisée en ce que** l'on peut obtenir l'enzyme à partir de souches du genre Lactobacillus.

7. Enzyme selon l'une des revendications précédentes que l'on peut obtenir à partir de Lactobacillus brevis (DSM 20054).

8. Alcool déshydrogénase stable, **caractérisée en ce que** l'enzyme présente essentiellement une séquence d'acides aminés selon le n° d'identification de séquence 8.

9. Alcool déshydrogénase stable, codée par une séquence d'ADN selon le n° d'identification de séquence 7 ou les analogues correspondants.

10. Procédé d'obtention de l'enzyme selon l'une des revendications 1 à 5, **caractérisé en ce qu'**un échantillon de micro-organismes éventuellement transformé avec un gène codant l'ADH est dissous et finalement soumis à une chromatographie d'interaction hydrophobe, une chromatographie d'affinité et d'échange d'anions dans des systèmes tampons appropriés contenant des ions magnésium.

11. Procédé selon la revendication 8, **caractérisé en ce que** le micro-organisme est le Lactobacillus brevis (DSM 20054).

12. Procédé de réduction énantiosélective de composés cétone organiques, **caractérisé en ce que** l'on utilise un composé de formule générale (I)

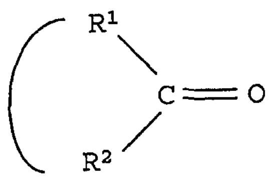

(I)

dans laquelle $R^1$ ou $R^2$, différents ou identiques, peuvent être un groupe hydrogène, ensemble ou isolément, un radical alkyle, alcènyle, aryle ou arylènyle, respectivement à chaîne ramifiée ou linéaire, constitué de 1 à 20 atomes de C, substitués par un ou plusieurs atomes d'halogène, des radicaux nitro, hydroxyle ou alcoxy, les radicaux alcoxy présentant 1 à 20 atomes de C, un groupe alkylène en $C_1$-$C_{10}$ éventuellement substitué, qui sont substitués par des hétérocycles azotés, oxygénés ou soufrés saturés, insaturés ou aromatiques, ou un radical polycondensé saturé et/ou aromatique éventuellement substitué, **caractérisé en ce qu'**un composé cétone ou un mélange correspondant est mis à incuber en présence d'une alcool déshydrogénase selon l'une des revendications 1 à 9 ou les cellules contenant l'enzyme entre environ 20° et 60°C sur une période d'environ 15 minutes à 3 heures et **en ce que** le composé R-hydroxy correspondant est isolé.

13. Procédé de synthèse énantiosélective de composés hydroxy organiques selon la formule générale (II)

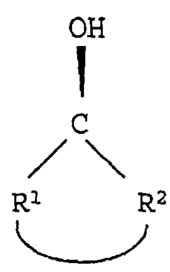

(II)

dans laquelle $R^1$ ou $R^2$, différents ou identiques, peuvent être un groupe hydrogène, ensemble ou isolément,

un radical alkyle, alcènyle, aryle ou arylènyle, respectivement à chaîne ramifiée ou linéaire, constitué de 1 à 20 atomes de C, substitués par un ou plusieurs atomes d'halogène, des radicaux nitro, hydroxyle ou alcoxy, les radicaux alcoxy présentant de 1 à 20 atomes de C, un groupe alkylène en $C_1$-$C_{10}$ éventuellement substitué, qui sont substitués par des hétérocycles d'azote, d'oxygène ou de soufre saturés, insaturés ou aromatiques, ou un radical polycondensé saturé et/ou aromatique éventuellement substitué, **caractérisé en ce qu'**un mélange racémique du composé hydroxy organique est mis à incuber avec une alcool déshydrogénase selon les revendications 1 à 9 ou avec les cellules contenant l'enzyme entre environ 20° et 60°C sur une période d'environ 15 minutes à 3 heures et **en ce que** le composé S-hydroxy correspondant est isolé.

**14.** Procédé selon la revendication 10 ou 11, **caractérisé en ce que** les ions magnésium sont présents et **en ce que** le pH se situe entre 6 et 9.

**15.** Procédé de préparation recombinante d'un alcool déshydrogénase stable, **caractérisé en ce que** la séquence de nucléotides selon le n° d'identification de séquence 7 est exprimée dans une cellule eucaryote ou procaryote.

**16.** Fragment d'ADN isolé, codant l'alcool déshydrogénase stable, **caractérisé en ce qu'**il contient une séquence d'acide nucléique selon le n° d'identification de séquence 7 ou une séquence hybridisante avec celle-ci.

**Abb. 1**

ATG TCT AAC CGT TTG GAT GGT AAG GTA GCA ATC ATT ACA GGT GGT ACG
M S N R L D G K V A I I T G G T
TTG GGT ATC GGT TTA GCT ATC GCC ACG AAG TTC GTT GAA GAA GGG
L G I G L A I A T K F V E E G
GCT AAG GTC ATG ATT ACC GGC CGG CAC AGC GAT GTT GGT GAA AAA
A K V M I T G R H S D V G E K
GCA GCT AAG AGT GTC GGC ACT CCT GAT CAG ATT CAA TTT TTC CAA CAT
A A K S V G T P D Q I Q F F Q H
GAT TCT TCC GAT GAA GAC GGC TGG ACG AAA TTA TTC GAT GCA ACG GAA
D S S D E D G W T K L F D A T E
AAA GCC TTT GGC CCA GTT TCT ACA TTA GTT AAT AAC GCT GGG ATC
K A F G P V S T L V N N A G I
GCG GTT AAC AAG AGT GTC GAA GAA ACC ACG ACT GCT GAA TGG CGT
A V N K S V E E T T T A E W R
AAA TTA TTA GCC GTC AAC CTT GAT GGT GTC TTC TTC GGT ACC CGA TTA
K L L A V N L D G V F F G T R L
GGG ATT CAA CGG ATG AAG AAC AAA GGC TTA GGG GCT TCC ATC ATC
G I Q R M K N K G L G A S I I
AAC ATG TCT TCG ATC GAA GGC TTT GTG GGT GAT CCT AGC TTA GGG GCT
N M S S I E G F V G D P S L G A
TAC AAC GCA TCT AAA GGG GCC GTA CGG ATT ATG TCC AAG TCA GCT
Y N A S K G A V R I M S K S A
GCC TTA GAT TGT GCC CTA AAG GAC TAC GAT GTT CGG GTA AAC ACT GTT
A L D C A L K D Y D V R V N T V
CAC CCT GGC TAC ATC AAG ACA CCA TTG GTT GAT GAC CTA CCA GGG
H P G Y I K T P L V D D L P G
GCC GAA GAA GCG ATG TCA CAA CGG ACC AAG ACG CCA ATG GGC CAT
A E E A M S Q R T K T P M G H
ATC GCT GAA CCT AAC GAT ATT GCC TAC ATC TGT GTT TAC TTG GCT TCT
I G E P N D I A Y I C V Y L A S
AAC GAA TCT AAA TTT GCA ACG GGT TCT GAA TTC GTA GTT GAC GGT GGC
N E S K F A T G S E F V V D G G
TAC ACT GCT CAA
Y T A Q

**Abb. 2**

T D R L K G K V A I V T G G T L G I G L A I A D K F V E E G A K V V I T G R H A
D V G E K -$X_{26}$- L F D A T E E A F G P V T T V V N N A G I A V N K S V E D T T T
E E W R K L L S V N L D G V F F G T R L G I Q A M K -$X_2$- G L G A S I I N M S S I
E G F V G D P T L G A Y N A S K G A V R I M S K S A A L D C A L K D Y D V R V
N T V H P G Y I K T P L V D D L E G A E E M M S Q R T K T P M G H I G E P N D
I A Y I C V Y L A S D E T K F A T G A E F V V D G G Y T A Q